(19) **Europäisches Patentamt / European Patent Office / Office européen des brevets**

(11) **EP 4 677 358 B1**

(12) # EUROPEAN PATENT SPECIFICATION

(45) Date of publication and mention
of the grant of the patent:
**20.05.2026  Bulletin 2026/21**

(21) Application number: **24754788.8**

(22) Date of filing: **17.07.2024**

(51) International Patent Classification (IPC):
**G01N 33/487** *(2006.01)*    **A61B 17/43** *(2006.01)*
**C12M 1/34** *(2006.01)*    **C12N 5/075** *(2010.01)*

(52) Cooperative Patent Classification (CPC):
**G01N 33/48728; A61B 17/43; C12M 41/46;
C12N 5/0609**

(86) International application number:
**PCT/TR2024/050832**

(87) International publication number:
**WO 2025/234954 (13.11.2025 Gazette 2025/46)**

(54) **METHOD FOR REAL-TIME DETERMINATION OF OOCYTE DEVELOPMENTAL COMPETENCE**

VERFAHREN ZUR ECHTZEITBESTIMMUNG DER ENTWICKLUNGSKOMPETENZ VON OOZYTEN

PROCÉDÉ DE DÉTERMINATION EN TEMPS RÉEL DE LA CAPACITÉ DÉVELOPPEMENTALE D'OVOCYTES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**14.01.2026  Bulletin 2026/03**

(73) Proprietor: **Çiray, Haydar Nadir
Rockville, Maryland 20850 (US)**

(72) Inventor: **Çiray, Haydar Nadir
Rockville, Maryland 20850 (US)**

(74) Representative: **Sögüt, Onur Ömer
Terra Patent Limited Sirketi
Hasanpasa Mahallesi
Nabizade Sokak, No: 82/A
Kadiköy
34722  Istanbul (TR)**

(56) References cited:
**US-A- 6 062 225    US-A1- 2004 182 707**

- **CHEN ZHONGRONG ET AL: "Sensing Cell Membrane Biophysical Properties for Detection of High Quality Human Oocytes", ACS SENSORS, vol. 4, no. 1, 25 December 2018 (2018-12-25), pages 192 - 199, XP093234073, ISSN: 2379-3694, DOI: 10.1021/ acssensors.8b01215**
- **PALAY PEYMAN ET AL: "Simple bioelectrical microsensor: oocyte quality prediction via membrane electrophysiological characterization", LAB ON A CHIP, vol. 24, no. 16, 29 June 2024 (2024-06-29), pages 3909 - 3929, XP093233972, ISSN: 1473-0197, DOI: 10.1039/ D3LC01120H**

## Description

### Technical Field

The present disclosure relates to assisted reproduction technologies (ART) such as in vitro fertilization (IVF).

### Background

During maturation, an oocyte accumulates molecules that are decisive on health of a subsequent embryo after merging with a sperm. Competence of an oocyte to establish a pregnancy even before fertilization is hereby considered in correlation with an extent of potential for supporting a subsequent embryo development to yield a viable pregnancy and eventually birth of a healthy newborn. To the best of our knowledge, prior art does not suggest any continuous, objective and real-time parameter or tool for prediction of such competency of the oocyte. Therefore, human ART operates through retrieving a high number of oocytes with random competencies from a woman and inseminating the oocytes to form as many embryos as possible and to identify the embryo with the most probability of establishing a pregnancy through subjective parameters. This treatment approach is not feasible since it causes wastage of many oocytes, which is a valuable biological material.

Some of the prior research approaches are disruptive for the utilized biological material (that is, oocytes) and others lack consistent data and clinical evidence, subjective and have safety and toxicity concerns. Main approaches available in such studies involve one or more of the following:

- conventional morphological assessment of the oocyte,
- assessment of oocyte morphology through application of artificial intelligence/machine learning,
- polarizing microscopy analysis of the spindle and the zona pellucida,
- uptake of vital dyes to determine intracellular activity of organelles,
- assessment of the metabolic turnover of the oocyte,
- molecular studies on genes and gene products obtained from the oocyte and the cumulus cells,
- di-electrophoretic separation according to the gravity of the oocyte,
- assessment of elastic properties of the oocyte through mechanical approaches.

As a publication related to assisted reproduction technologies, WO 2020/077244 A1 is entirely focused on determination of oocyte viability as quality of oocyte.

Palay et al., Lab on a Chip, vol. 24 (2024), pages 3909-3929, discloses benchmarking of oocytes based on a measured electrical impedance, the measurement being performed using electrodes at the bottom of a well that contains an electrolyte bath, into which the oocyte is placed.

Hence, a method with a higher extent of accuracy is still sought in real-time benchmarking of oocytes for enhancing success rates in assisted reproduction technologies.

### Summary

The present disclosure aims to increase the efficiency of ART through establishing quantitative and real-time parameters in order to predict oocyte competency before it is inseminated, without compromising the utilization of the assessed oocyte for subsequent treatment. The present technique is unique as opposed to many approaches in the related field, by enabling quality assessment of oocytes, before stripping the same of their surrounding cells which support their maturation through passage of molecules thereinto. It is hereby disclosed a method for benchmarking of two or more mammalian oocytes in terms of their comparative extents of potential for supporting subsequent embryo development relative to one another.

Within the context of the present disclosure, different from the viability status of the oocyte; the term oocyte quality or quality of an oocyte refers to developmental competence, in other words, extent of potential for supporting a subsequent embryo development to yield a viable pregnancy and eventually birth of a healthy newborn. In particular, the present disclosure relates to an instant determination of oocyte developmental competence in real-time by assessment of its plasma membrane permeability and its cellular resistance. The technique proposed herein is further advantageous over various prior approaches as it provides real-time and continuous data on the quality of the oocyte. This is important as the oocyte quality deteriorates within few hours after ovulation and it is not possible to detect/monitor such changes since they do not reflect to the morphology of the cell.

Because the proposed technique will establish objective criteria to predict oocyte quality, ART treatment will develop or improve strategies which will target producing oocytes with high competencies. Also, many couples currently defined as "difficult cases" will benefit and will have a probability of achieving success from the ART treatment without sacrificing of a high number of oocytes. Furthermore, oocyte banking will be able to store gametes with known competencies and in-vitro oocyte maturation studies will be able to utilize these parameters as a guide in optimizing their methods.

In a first aspect of the invention, the proposed method comprises the following steps:

step-A) determination of relative extents of respective plasma membrane permeabilities of a first oocyte and a second oocyte with regard to one another, and

step-B) sorting the first oocyte and the second oocyte in accordance with their relative extents of respective plasma membrane permeabilities, such that one of the first oocyte and the second oocyte with a relatively lower plasma-membrane permeability is benchmarked as having a relatively greater extent of potential for supporting a subsequent embryo development.

The determination of relative extents of respective membrane permeabilities of the first oocyte and the second oocyte in the step-A is based on one or more measurements of relative extents of one or more electrical parameters of the first oocyte and the second oocyte, respectively. Electrical resistance can be selected as the one or more electrical parameters and can be calculated from one or more potential difference measurements.

The step-A comprises the following steps:

i) placement of the first oocyte into an electrolyte bath;
ii) when a first electrode is immersed into the electrolyte bath, and a second electrode is engaged with the first oocyte, provision of an electrical current between the first electrode and second electrode by a current source, such that the electrical current flows through the electrolyte and a membrane of the first oocyte; measuring and recording a first potential difference change value in relation with the first oocyte;
iii) placement of the second oocyte into the electrolyte bath;
iv) when the first electrode is immersed into the electrolyte bath, and the second electrode is engaged with the second oocyte, provision of the electrical current between the first electrode and second electrode by the current source, such that the electrical current flows through the electrolyte and a membrane of the second oocyte; measuring and recording a second potential difference change value in relation with the second oocyte;

and the step-B comprises the following:
v) if the first potential difference change is smaller than the second potential difference change, benchmarking the second oocyte as having a relatively greater extent of potential for supporting subsequent embryo development; or if the first potential difference change is greater than the second potential difference change, benchmarking the second oocyte as having a relatively lower extent of potential for supporting subsequent embryo development.

Within the context of the present disclosure, engagement between the second electrode with an oocyte (the first oocyte or a further oocyte such as the second oocyte) corresponds to that the second electrode (e.g., a tip thereof) is brought into electrical communication with an intracellular medium of the respective oocyte, for instance, by impaling of the respective oocyte, in other words, insertion of the second electrode (e.g., the tip thereof) into the respective oocyte.

The step ii can comprise calculation of a first resistance value based on the first potential difference change and attributing the first resistance value as a membrane resistance of the first oocyte. Likewise, the step iv can comprise calculation of a second resistance value based on the second potential difference change and attributing the second resistance value as a membrane resistance of the second oocyte.

For obtaining a baseline potential difference, the method can comprise the following step-x prior to the step-ii: x) measurement of a potential difference between the first electrode and the second electrode that are immersed into the electrolyte bath in the absence of the electrical current between the first electrode and second electrode by the current source.

The step ii and/or step iv can be followed by extraction of the second electrode from the first oocyte or second oocyte, and measuring potential difference for confirming whether the baseline potential difference is maintained in the absence of the electrical current between the first electrode and second electrode by the current source. If this potential difference measurement does not deviate more than 10% from the baseline potential difference (on the basis of the originally measured or a former value of baseline potential difference), it can be confirmed that the second electrode is still functional and the recorded/monitored/tracked parameters are accurate.

In order to confirm a viability status for the first oocyte, the method can comprise the following:
when the second electrode is inserted into the first oocyte, measuring a cell potential difference for the first oocyte (that is, a fourth potential difference in the absence of electrical current feed by the current source between the first electrode and second electrode); checking whether a difference between the baseline potential difference and the cell potential (i.e., fourth potential difference) is different from zero and does not deviate more than 10% (e.g., more than 2 mV) on the basis of an initial absolute value thereof (e.g., 20 mV), throughout (or at the end of) a first duration for the first oocyte (e.g., 30 seconds or more) for confirming a viability status for the first oocyte.

Likewise, viability status for the second oocyte can be confirmed by the following actions:

when the second electrode is inserted into the second oocyte, measuring a cell potential for the second oocyte (that is, the fourth potential difference in the absence of electrical current feed by the current source between the first electrode and second electrode); checking whether the difference between the baseline potential difference and the cell potential difference (that is, fourth potential difference) is different from zero and does not deviate more than 10% (e.g., more than 2 mV) on the basis of an initial absolute value thereof (e.g., 20 mV), throughout (or at the end of) a respective first duration for the second oocyte (e.g., 30 seconds or more) for confirming an initial viability status for the second oocyte.

Any version of the proposed method can comprise removal of one or more cumulus cells from the respective oocyte (e.g., first oocyte and/or second oocyte) prior to the step-A.

In a second aspect of the invention, the step-A of the proposed method comprises the following steps:

i) placement of the first oocyte into the electrolyte bath;

ii) bringing the first electrode into electrical communication with the electrolyte bath, and bringing a second electrode into electrical communication with an intracellular medium of the first oocyte, provision an electrical current (through the second electrode, or) between the first electrode and second electrode by a current source, such that the electrical current flows through a membrane of the first oocyte; measuring and recording a first potential difference change value in relation with the first oocyte; calculation of a first resistance value based on the first potential difference change, and attributing the first resistance value as a membrane resistance in relation with the first oocyte;

and the step-B comprises the following:

iii) determining whether the respective first resistance value in relation with the first oocyte is higher than a pre-determined reference value; and if the first resistance value is greater than the reference value, benchmarking the first oocyte as having a sufficient extent of potential for supporting subsequent embryo development.

**Brief Description of the Drawings**

FIG. 1A schematically shows an experimental setup at a stage for determination of the baseline potential difference without provision of electrical current by a current source.

FIG. 1B schematically shows a stage for determination of resistance of the second electrode in the presence of current provision by the current source.

FIG. 1C schematically shows a stage in which the second electrode is engaged with the first oocyte, in the absence of current provision by the current source, for measuring a fourth potential difference.

FIG. 1D schematically shows a stage in which the second electrode is engaged with the first oocyte, in the presence of current provision by the current source, for measuring a first potential difference.

FIG. 2A shows repetition of the action schematized in FIG. 1A for confirming the baseline potential difference at preparation for, or after completion of measurements relevant to the second oocyte.

FIG. 2B schematically shows confirmation of that the electrode resistance is maintained prior to, or after completion of measurements relevant to the second oocyte, as in FIG. 1B.

FIG. 2C schematizes a stage for determination of viability status of the further oocyte over the respective fourth potential difference, as in FIG. 1C.

FIG. 2D schematizes a stage for determination of plasma-membrane permeability of the further oocyte as in FIG. 1D, over the respective first potential difference.

FIG. 3A represents electrical parameter measurements consecutively applied for two different oocytes.

FIG. 3B represents electrical parameter measurements consecutively applied for two different oocytes.

FIG. 4 visualizes a basic scheme regarding the hypothesis of the proposed technology.

FIG. 5A shows microscopic images of oocytes which have been stripped of (left) and enclosed in their cumulus investments (right). The stripped oocyte is secured in place with a holding pipette and approached by a tip of an exemplary embodiment of a second electrode.

FIG. 5B schematizes injection of electrical current into a first oocyte (left) with a relatively higher plasma membrane permeability (dashed circle); and into a further or second oocyte (right) with a relatively lower plasma membrane permeability (solid circle).

FIG. 5C symbolizes consecutive electrical potential difference recordings corresponding to the two oocytes depicted in FIG. 5B, respectively.

FIG. 6 relates to consecutive recordings for two oocytes; here, the oocytes are denuded.

FIG. 7A shows proof of principle pilot data in milliVolts, on denuded bovine oocytes showing changes in membrane potential difference during maturation from Prophase 1 (GV) to Metaphase I (MI) and II (MII).

FIG. 7B, with reference to FIG. 7A, shows proof of principle pilot data in MegaOhms, on denuded bovine oocytes showing changes in input resistance during maturation from Prophase 1 (GV) to Metaphase I (MI) and II (MII).

FIG. 7C shows comparison in electrophysiological parameters (in milliVolts) of arrested oocytes at GV stage, to corresponding Day 1 oocytes.

FIG. 7D show comparison in electrophysiological parameters (in MegaOhms) of arrested oocytes at GV stage, to corresponding Day 1 oocytes.

FIG. 7E shows comparison in electrophysiological parameters (in milliVolts) of arrested oocytes at MI stage, to corresponding Day 1 oocytes.

FIG. 7F show comparisons in electrophysiological parameters (in MegaOhms) of arrested oocytes at MI stage, to corresponding Day 1 oocytes.

FIG. 8 shows electrical parameters profile of 111 metaphase II bovine oocytes.

FIG. 9A shows proportion of mouse zygotes that yielded blastocysts after thawing (control, n=55), followed by cellular impalements without (MP, n=51) and with (MPIR, n=56) current injections ($P$=0.19, one-way ANOVA).

FIG. 9B in relation with FIG. 9A, shows total cell counts of blastocysts from the control (n=11), MP (n=7) and MPIR (n=6) groups (P=0.92, one-way ANOVA).

<u>Reference signs</u>

| | |
|---|---|
| 1 | bath |
| 11 | first electrode |
| 12 | second electrode |
| 51 | first oocyte |
| 510 | membrane |
| 52 | second oocyte |
| 520 | membrane |
| 521 | cumulus cells |
| 7 | electrical monitoring and control system |
| 70 | measuring device |
| 71 | current source |
| 72 | switch |
| 100 | setup |
| A | membrane potential difference |
| A1 | axis |
| B | input resistance |
| C | maturity |
| D | competency |
| I | current provided by the current source |
| O1 | test period for the first oocyte |
| O2 | test period for the second oocyte |
| t1 | first duration for the first oocyte |
| t1' | first duration for the second or further oocyte |
| t2 | second duration for the first oocyte |
| t2' | second duration for the second or further oocyte |
| t0 | non-engagement duration for the first oocyte |
| t0' | non-engagement duration for the second oocyte |
| t51 | extra-oocyte current injection duration |
| t52 | extra-oocyte current injection duration |
| t53 | extra-oocyte current injection duration |
| t54 | extra-oocyte current injection duration |
| U0 | baseline potential difference |
| U1 | first potential difference |
| U2 | second potential difference |
| Ue | second electrode's potential difference |
| Um | fourth potential difference |

Um1    fourth potential difference for the first oocyte
Um2    fourth potential difference for the second (or, further) oocyte
$\Delta$U1    first potential difference change
$\Delta$U2    second potential difference change

**Detailed description**

The proposed technique hypothesizes that plasma membrane permeability changes and modifications in cytoplasmic characteristics that occur during maturation are correlated to the developmental competency of the oocyte. These alterations are herein reflected to electrophysiological parameters (EP) of the oocyte as changes in the membrane potential difference (Um) and the (cellular and/or cytoplasmic) input resistance (IR), thereby enabling the monitoring of the oocyte developmental competency in real time, without compromising cellular viability or capacity to support embryo development. The cellular and/or cytoplasmic input resistance (IR) values are calculable from respective potential difference changes ($\Delta$U1 and $\Delta$U2).

Plasma membrane permeability of an oocyte decreases throughout the maturation process thereof due to gradually ceasing intercellular communication among the oocyte and the surrounding cumulus cells. This reflects to an increase of membrane resistance (that is, plasma-membrane resistance) of the oocyte. The present disclosure aims to benchmark oocytes in view of their extent of progress in maturation relative to one another. Within the context of the present disclosure, the extent of progress in maturation is considered relevant to an extent of potential for supporting a subsequent embryo development.

Accordingly, respective membrane resistances of two or more oocytes are measured, or, observed, relative to one another; and one of the oocytes with a comparatively high extent of membrane resistance is benchmarked as having a relatively higher extent of potential for supporting a subsequent embryo development, in comparison with another one of the oocytes that is subjected to the same measurement, or in comparison with the rest of oocytes if there are more than two oocytes that are subjected to the same measurement. It can be inherently considered that the term "same measurement" corresponds to that all of the parameters (other than the oocytes being different from one another) are maintained the same, or at least intended to do so as precisely as possible.

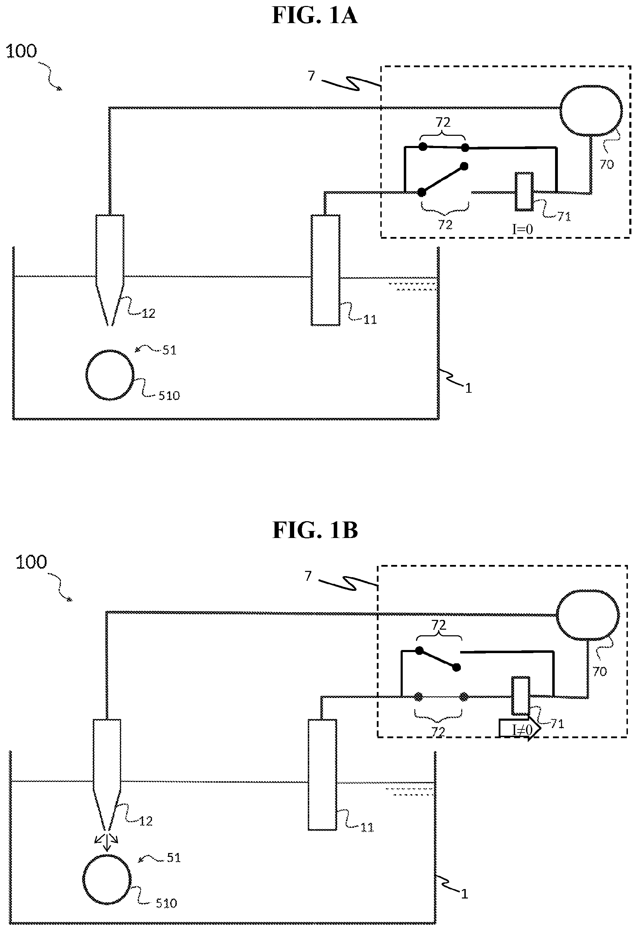

FIG. 1A schematically shows an experimental setup (100) at a stage for determination of the baseline potential difference (U0) without provision of electrical current (I=0) by a current source (71). The circuit is closed by electrically communicating the first electrode (11) and second electrode (12) through the electrolyte bath (1). In the absence of current provision (I=0) by the current source (71); ionic composition difference between electrolyte bath (1) and second electrode (12) results in the baseline potential difference (U0).

FIG. 1B schematically shows a stage for determination of resistance of the second electrode (i.e., electrode resistance, calculable from second electrode's potential difference Ue) in the presence of current provision (I$\neq$0 Amperes) by the current source (71). The smaller a tip opening diameter of the second electrode (12), the greater the electrode resistance, reflecting into a greater extent of absolute value of second electrode's potential difference (Ue). In the relevant drawings, presence of current feed through the second electrode (12) is symbolized with arrows radiating from the tip of the second electrode (12).

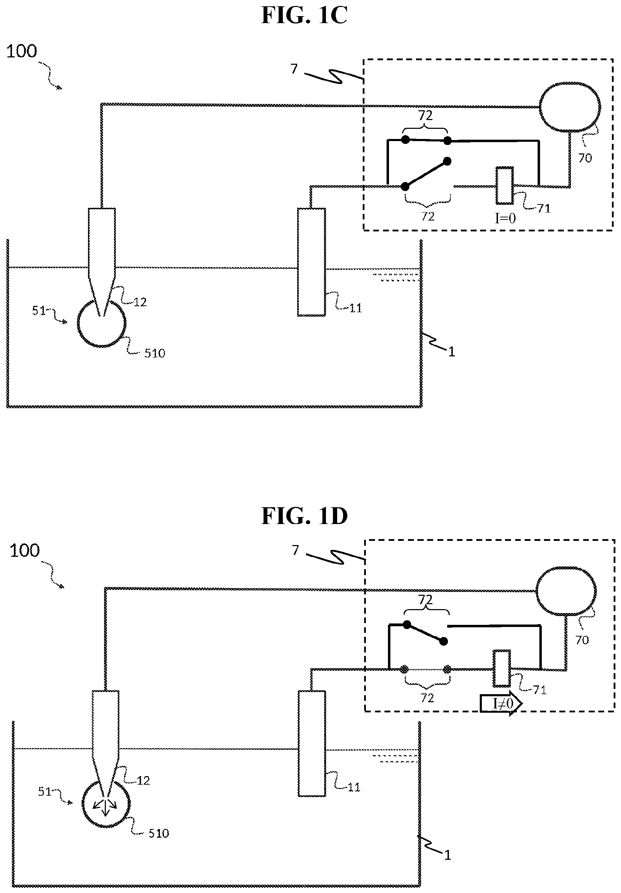

FIG. 1C schematically shows a stage in which the second electrode (12) is engaged with the first oocyte (51), in the absence of current provision (I=0) by the current source (71). Confirmation of the engagement with the respective oocyte is achieved by observing an alteration of the measured potential difference, such that the potential difference drops by fourth potential difference (Um, Um1) (here, that for the first oocyte (51)), due to a barrier formed by the plasma-membrane (510) of the respective oocyte between inside and the outside of the cell (here, first oocyte (51)) creating the potential difference drop related to the variations of the ionic compositions. Since a non-viable oocyte plasma membrane (here, 510) would lose its capacity to form the barrier, hence exhibit a reduced and/or no potential difference drop; this measurement also serves for determination of viability status for the respective oocyte (here, first oocyte 51).

FIG. 1D schematically shows a stage in which the second electrode (12) is engaged with the first oocyte (51), in the presence of current provision (I$\neq$0 Amperes) by the current source (71). The measured potential difference drops by a first potential difference change ($\Delta$U1) (with regard to the fourth potential difference Um1), thereby culminating in a value of a first potential difference (U1). Smaller absolute values of the first potential difference change ($\Delta$U1) indicates that the respective oocyte (here, first oocyte 51) has a lower plasma-membrane permeability corresponding to a lower level of oocyte maturation. Hence, higher plasma membrane permeability corresponds to a lower level of oocyte maturation.

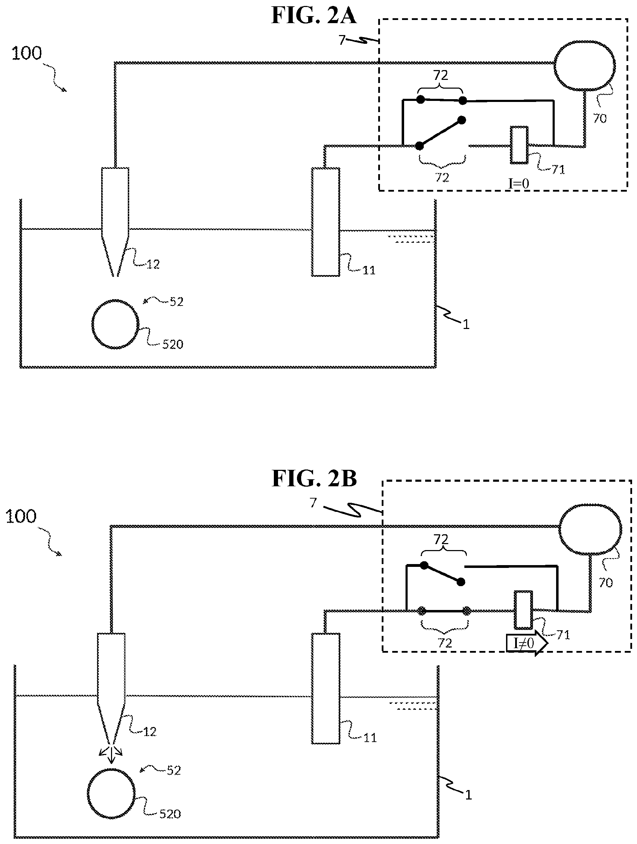

FIG. 2A shows repetition of the action schematized in FIG. 1A for confirming the baseline potential difference (U0) at preparation for, or after completion of measurements relevant to the second oocyte (52).

FIG. 2B schematically shows confirmation of that the electrode resistance is maintained prior to, or after completion of measurements relevant to the second oocyte, as in FIG. 1B. Dramatic changes in the electrode resistance which inherently reflect into dramatic deviations from the initial value of the second electrode's potential difference (Ue) (e.g., greater than 10% in original absolute value thereof which is measured earlier with reference to FIG. 1B) would implicate that the second electrode (12) may be damaged.

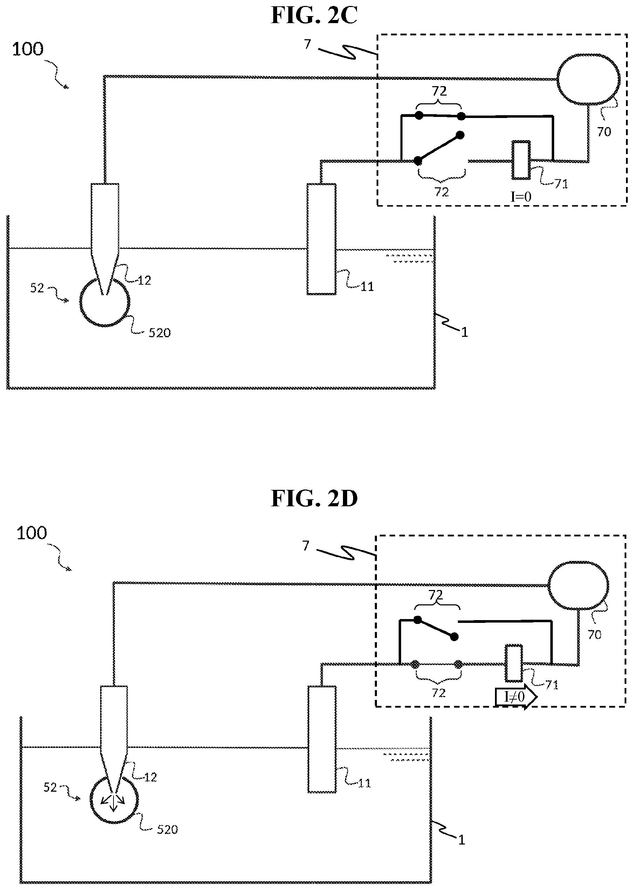

FIG. 2C schematizes a stage for determination of viability status of the further oocyte (here, second oocyte 52), as in FIG. 1C.

FIG. 2D schematizes a stage for determination of plasma-membrane permeability of the further oocyte (here, second oocyte 52), as in FIG. 1D.

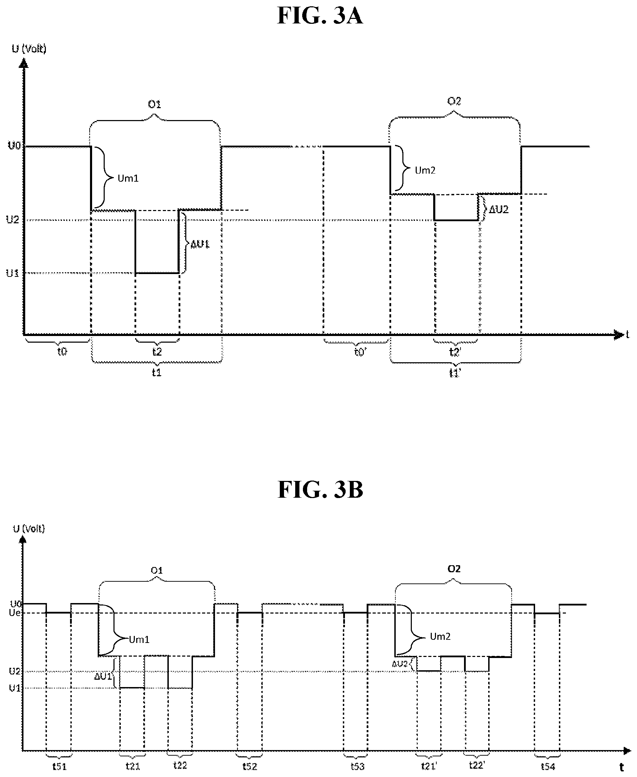

FIG. 3A represents electrical parameter (here, voltage, in other words, potential difference) measurements consecutively applied for two different oocytes. Non-engagement durations (t0 and t0') correspond to the stages depicted in FIG. 1A and FIG. 2A, respectively; which are prior to engaging the second electrode (12) with the first oocyte (51) and second oocyte (52); here, the voltage measurement corresponds to baseline potential difference (U0). First durations (t1 and t1') correspond to those at which the second electrode (12) is engaged to first oocyte (51) and second oocyte (52), respectively. With reference to FIG. 1D and FIG. 2D, second durations (t2 and t2') correspond to instances in which electrical current is provided (I≠0 Amperes) by the current source (71) and injected through the second electrode (12) into the first oocyte (51) and second oocyte (52), respectively. Within the respective first durations (t1 and t1') but outside the second durations (t2 and t2'), no electrical current is provided (I=0) by the power source (71) and fourth potential difference values (Um1 and Um2) are obtained for the first oocyte (51) and second oocyte (52), respectively; such instances are depicted in FIG. 1C and FIG. 2C.

FIG. 3B represents electrical parameter (here, voltage) measurements consecutively applied for two different oocytes. Extra-oocyte current injection durations t51 and t52 correspond to a stage depicted in FIG: 1B, whereas the extra-oocyte current injection durations t53 and t54 correspond to a stage depicted in FIG. 2B, at presence of current (I≠0) is provided by the current source (72); which are prior to engaging (or after ceasing the engagement of) the second electrode (12) with the first oocyte (51) and second oocyte (52). Here, the voltage drops from the baseline potential difference (U0) imply the resistance of the second electrode (calculable from the second electrode's potential difference (Ue)) (12).

Oocyte test periods (O1 and O2) represent the periods at which the second electrode (12) is engaged to first oocyte (51) and second oocyte (52), respectively. Here, injection of current (I≠0) into the respective oocytes are applied, preferably up to two times per oocyte, for monitoring the respective stabilities (or in other words, consistencies) of first potential difference change (ΔU1) and second potential difference change (ΔU2) measurements.

With reference to FIG. 3A, if the respective fourth potential difference values (Um1 and Um2) shift throughout the relevant first durations (t1 and t1') more than 10% (based on their initial values measured at beginning of respective first durations t1 and t1'), it can be determined that the respective first oocyte (51) or second oocyte (52) is no longer viable.

Accordingly; the method can comprise the following

- when the second electrode (11) is engaged to the first oocyte (51), measuring a fourth potential difference (Um, here: Um1) in the absence of electrical current (I=0) feed by the current source (71) between the first electrode (11) and second electrode (12); checking whether a difference between the baseline potential difference (U0) and fourth potential difference (Um or Um1, here: Um1) is different from zero (e.g., -20 mV, that is 20 mV of drop in potential difference upon engaging the first oocyte 51) and does not deviate more than 10% on the basis of an initial absolute value thereof (e.g., 2 mV or less deviation for 20 mV absolute value of Um1-U0), at the end of a first duration for the first oocyte (t1) (e.g., 30 seconds or more) for confirming viability status for the first oocyte (51).

Likewise; the method can comprise the following:

- when the second electrode (11) is engaged to the second oocyte (52), measuring the fourth potential difference (Um, here: Um2) in the absence of electrical current (I=0) feed by the current source (71) between the first electrode (11) and second electrode (12); checking whether the difference between the baseline potential difference (U0) and fourth potential difference (Um or Um2, here: Um2) is different from zero (e.g., -20 mV, that is 20 mV of drop in potential difference upon engaging the second oocyte 52) and does not deviate more than 10% on the basis of an initial absolute value thereof (e.g., 2 mV or less deviation for 20 mV absolute value of Um2-U0), at the end of a first duration for the second oocyte (t1') (e.g., 30 seconds or more) for confirming a viability status for the second oocyte (52).

With reference to FIG. 1D and FIG. 2D in conjunction with FIG. 3B, second durations (t21 and t22 for FIG. 1D; t21' and t22' for FIG. 2D) correspond to instances in which electrical current is provided (I≠0 Amperes) by the current source (71) and injected through the second electrode (12) into the first oocyte (51) and second oocyte (52), respectively. Within the respective oocyte test periods (O1 and O2) but outside the second durations (t21, t22, t21' and t22'), no electrical current is provided (I=0) by the power source (71) and fourth potential difference values (Um1 and Um2) are obtained for the first oocyte (51) and second oocyte (52), respectively; such instances are depicted in FIG. 1C and FIG. 2C.

Referring to the appended drawings, the present disclosure proposes a method for benchmarking two or more mammalian oocytes in terms of their comparative extents of potential for supporting subsequent embryo development relative to one another. The method comprises the following steps:

step-A) determination of relative extents of respective plasma membrane permeabilities of a first oocyte (51) and a second oocyte (52) with regard to one another, and

step-B) sorting the first oocyte (51) and the second oocyte (52) in accordance with their relative extents of respective plasma membrane permeabilities, such that one of the first oocyte (51) and the second oocyte (52) with a relatively lower plasma-membrane permeability is benchmarked as having a relatively greater extent of potential for supporting subsequent embryo development.

Within the context of the present disclosure, one or more further oocytes can be consecutively tested as it is performed for the second oocyte (52), so that sorting of a higher number of oocytes in terms of their relative potentials for supporting respective embryo developments can be achieved relying on the main concept that is disclosed herein.

The determination of relative extents of respective plasma-membrane permeabilities of the first oocyte (51) and the second oocyte (52) in the step-A is based on one or more measurements of relative extents of one or more electrical parameters of the first oocyte (51) and the second oocyte (52), respectively. The one or more electrical parameters involve potential difference (i.e., voltage) measurements, and resistance values can be calculated based on said potential difference measurements. Thus, the method can comprise the use of electrical resistance as the one or more electrical parameters.

Referring to FIG. 1D and FIG. 3A; in a first version of the method, the step-A comprises the following steps:

- placement of the first oocyte (51) into an electrolyte bath (1);
- when a first electrode (11) is immersed into the electrolyte bath (1), and a second electrode (12) is inserted into the first oocyte (51), providing an electrical current ($I \neq 0$ Amperes) between the first electrode (11) and second electrode (12) by a current source (71), such that the electrical current flows through the electrolyte and a membrane (510) of the first oocyte (51); measuring and recording a first potential difference change ($\Delta U1$) value in relation with the first oocyte (51); calculation of a first resistance value based on the first potential difference change ($\Delta U1$), and attributing the first resistance value as a membrane resistance in relation with the first oocyte (51).

In the first version, the step-B comprises the following:

- determining whether the respective first resistance value in relation with the first oocyte (51) is higher than a pre-determined reference value; and if the first resistance value is greater than the reference value, benchmarking the first oocyte (51) as having a sufficient extent of potential for supporting subsequent embryo development.

The first version of the method may be considered to require completion of a series of third-phase experiments for determining the reference value. On the other hand, the following comparative approach for benchmarking in terms of relative potentials for supporting subsequent embryo development, eliminates such requirement related to the third-phase experiments.

Accordingly, with reference to FIG. 1A to FIG. 3B, in particular, to FIG. 1C, FIG. 2C in conjunction with FIG. 3A; in a second version of the method, the step-A comprises the following steps:

(i) placement of the first oocyte (51) into the electrolyte bath (1);

(ii) when the first electrode (11) is immersed into the electrolyte bath (1), and a second electrode (12) is engaged to the first oocyte (51), providing an electrical current ($I \neq 0$ Amperes) between the first electrode (11) and second electrode (12) by the current source (71), such that the electrical current flows through the electrolyte and a membrane (510) of the first oocyte (51); measuring and recording a first potential difference change ($\Delta U1$) value in relation with the first oocyte (51); which can be followed by a calculation of a first resistance value based on the first potential difference change ($\Delta U1$), and attributing the first resistance value as a membrane resistance of the first oocyte (51); (see FIG. 1C in conjunction with FIG. 3A)

(iii) placement of the second oocyte (52) into the electrolyte bath (1);

(iv) when the first electrode (11) is immersed into the electrolyte bath (1), and a second electrode (12) is engaged to the second oocyte (52), providing the electrical current ($I \neq 0$ Amperes) between the first electrode (11) and second electrode (12) by the current source (71), such that the electrical current flows through the electrolyte and a membrane (520) of the second oocyte (52); measuring and recording a second potential difference change ($\Delta U2$) value in relation with the second oocyte (52); which can be followed by a calculation of a second resistance value based on the second potential difference change ($\Delta U2$), and attributing the second resistance value as a membrane resistance of the second oocyte (52) (see FIG. 2C in conjunction with FIG. 3A).

The step-B comprises the following:

(v) if the first potential difference change (ΔU1) is smaller than the second potential difference change (ΔU2), benchmarking the first oocyte (51) as having a relatively lower extent of potential for supporting subsequent embryo development; or if the first potential difference change (ΔU1) is greater than the second potential difference change (ΔU2), benchmarking the second oocyte (52) as having a relatively lower extent of potential for supporting subsequent embryo development.

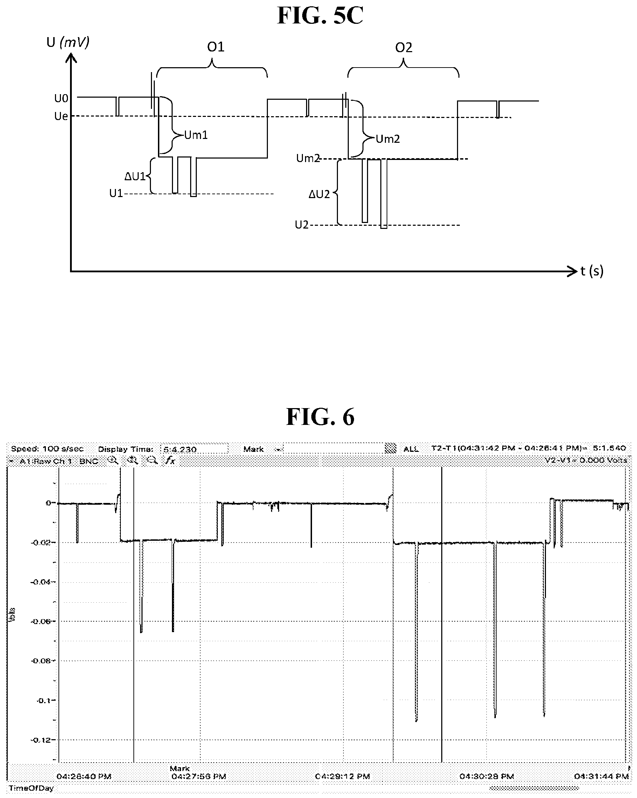

The step-iii can be applied before or after the step-ii. The step-iii and step-iv can be respectively considered as repetitions of step-ii, mutatis mutandis; that is, other than that the first oocyte (51) and second oocyte (52) are different from one another, all of the parameters relevant to formation and operation of the setup (100) are maintained. The setup (100) includes the followings: the electrolyte bath (1), first electrode (11), second electrode (12), along with an electrical monitoring and control system (7) for monitoring potential difference values obtained from the first and second electrodes in an amplified fashion, comprising one or more electrometers (70) (that can be, e.g., Avometer, ampermeter and/or voltmeter), one or more current supplies (71) for provision of the electrical current (I) and one or more switches (72) for closing a circuit along said elements or for selectively by-passing the one or more current supplies (71). Momentary states of an exemplary configuration of the electrical monitoring and control system (7) at different steps of the present method is schematized in FIG. 1A to FIG. 2D. The electrical monitoring and control system (7) can be connected to one or more displays for visual presentation of relevant information as in FIG. 6. The electrical monitoring and control system (7) can be connected to one or more computers, e.g., for simultaneous calculation of electrical resistances from momentary values of potential difference.

In the second version, the step-B can comprise the following:

B1) determining which one of the first resistance value and second resistance value is relatively higher;
B2) benchmarking the first oocyte (51) as having a relatively greater extent of potential for supporting subsequent embryo development, if the first resistance value is higher than the second resistance value; or benchmarking the second oocyte (52) as having a relatively greater extent of potential for supporting subsequent embryo development, if the second resistance value is higher than the first resistance value.

With reference to FIG. 1A and FIG. 2A in conjunction with FIG. 3A and FIG. 3B; any of the first version or second version of the method can comprise the following step-x, e.g., prior to the step-(ii):
x) when the first electrode (11) and second electrode (12) are immersed into the electrolyte bath (1), without engaging the second electrode (12) with a respective oocyte and without feeding an electric current (I=0) by the current source (71), measuring a baseline potential difference (U0) between the first electrode (11) and the second electrode (12).

The first electrode (11) can be considered as a reference electrode, whereas the second electrode (12) can be referred to as a microelectrode that can be used in ART. Such microelectrode can be considered to comprise a hollow body with a tapered tip having an opening for being engaged to an oocyte (e.g., at step-ii or step-iv). The body can be provided with an ionic solution with a composition that can be different from the electrolyte of the electrolyte bath (1), such that the second electrode (12) has an electrode potential difference due to migration of ions through the opening, between the hollow body and electrolyte bath (1).

A baseline resistance value can be calculated from the baseline potential difference (U0) and can be attributed as an electrode resistance of the second electrode (12) (hereinafter briefly referred to as electrode resistance).

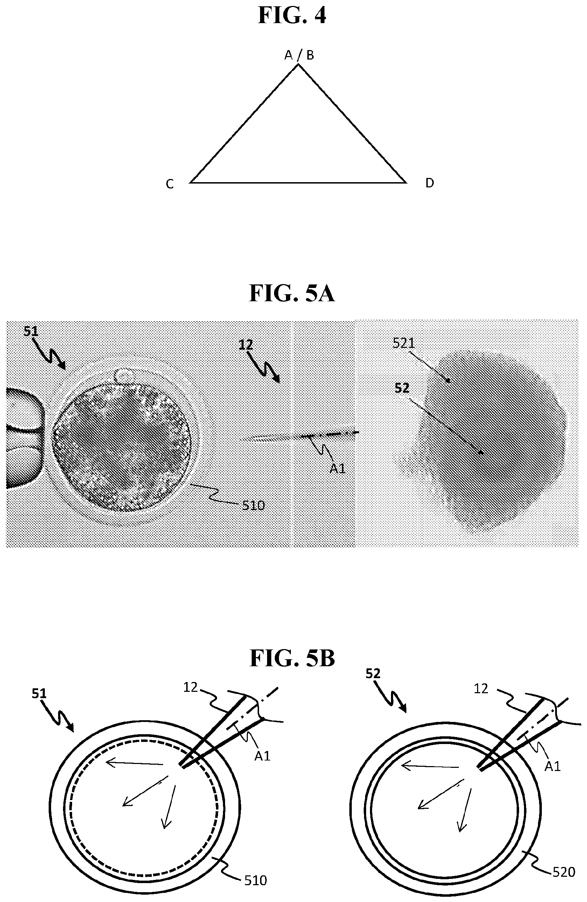

It can be considered that the electrode resistance would have a lower value with a greater extent of tip opening (that is, cross-sectional area across an electrical current flow direction therethrough, or across a main axis (A1) of the second electrode (12), see FIG. 5A to FIG. 5C). A small value of electrode resistance may be desired for reduction of possible noise in obtaining voltage data. On the other hand, the second electrode (12) is preferably to be selected to have a smaller tip opening (thus, the size of the tip), for enhancing the chance of engaging with oocytes without mechanically damaging the same. Smaller extents of tip opening (e.g., diameter or cross-section thereof across the main axis (A1)) inherently result in greater extents of the respective electrode resistance; hence, a sweet spot can be sought. Within this sense, the present disclosure gives a reproducible example that enables sufficiently low-noise data without damaging oocytes. In these terms, considering the value of current provided by the current source (71) and compositions of electrolytes inside the bath (1) and second electrode (12), electrode resistances within the range of e.g., 20 MegaOhms and 50 MegaOhms are considered highly useful and available, in other words, arrangeable.

In a version in which the step-x applies prior to the step-ii, the step-ii can be followed by extraction of the second electrode (12) from the respective one of the first oocyte (51) or second oocyte (52), repeating the step-ii for confirming whether the baseline potential difference (U0) (thus, the baseline resistance value) is maintained (see FIG. 3B).

Referring back to FIG. 3A in conjunction with FIG. 1C or FIG. 2C, any of the first and second versions of the method can comprise the following feature:
when the second electrode (11) is inserted into the respective one of the first oocyte (51) or second oocyte (52), measuring a fourth potential difference (Um) (that is, respective plasma membrane potential difference) in the absence of electrical current feed (I=0) by the current source (71) between the first electrode (11) and second electrode (12); checking whether the difference between the baseline potential difference (U0) and fourth potential difference (Um) is different from zero Volt (thus, negative milliVolts) and does not deviate more than 10% on the basis of an initial absolute value thereof, at the end of a first duration for the first oocyte (t1) for confirming a viability status for the first oocyte (51), for confirming a viability status for the respective one of the first oocyte (51) or second oocyte (52).

For instance;

- if the baseline potential difference (U0) and fourth potential difference (Um, or Um1 for the first oocyte 51 and Um2 for the second oocyte 52) are far from one another, e.g., if the difference therebetween is not substantially less than 15-20 mV at system parameters (such as ion concentrations in the second electrode 12 and in the bath 1) exemplified in EXAMPLES section of the present specification, the respective oocyte can be considered viable.

FIG. 4 visualizes a basic scheme regarding the hypothesis of the proposed technology. A refers to membrane potential difference (RMP or Um), B is input resistance (IR), C is oocyte maturity and D is oocyte developmental competency.

FIG. 5A shows microscopic images of oocytes which have been stripped of (left, a first oocyte 51) and enclosed in (right, a further oocyte 52) their cumulus investments (that is, cumulus cells 521). Here, the stripped, first oocyte (51) is secured in place with a holding pipette and approached by a tip of the second electrode (12), along the axis (A1) thereof. Within the context of the present disclosure, the second electrode (12) can be considered as an intracellular sharp electrode that can be used in oocyte-relevant electrical measurements.

FIG. 5B schematizes the state for obtaining a stable membrane potential difference recording, (e.g., 1 nA) current (symbolized with radially extending arrows) is passed from the second electrode (12) into a first oocyte (51) with a relatively high (left, represented using a dashed inner ring) membrane (510) permeability and into a second oocyte (52) with a relatively low (right, represented using a solid inner ring) membrane (520) permeability.

Any version of the method can comprise, prior to the step step-A, removal of a respective cumulus cells (521) from the first oocyte (51) and/or from the second oocyte (52) (see FIG. 5A).

EXAMPLES

The proposed technology can be applied by a series of experiments, thereby developing objective tools for predicting a relative capacity of an oocyte to support a viable pregnancy in real-time, without compromising subsequent utilisation thereof. The following examples merely intend provision of a better understanding with regard to the proposed technology, without unduly limiting the intended scope of protection.

EXAMPLE 1

Second electrode: As second electrode (12), a borosilicate glass pipette which can preferably pulled to fine tips suitable for provision of an electrode resistance within the range between 20 and 50 Mega Ohms (M$\Omega$), can be provided and filled with an ionic solution such as an aqueous solution of KCl with a concentration of 3 moles per Liter. The second electrode (12) can also be referred to as microelectrode. The microelectrode is connected to an electrometer, e.g., through an Ag/AgCl wire; and a circuit can be established by placing a reference electrode (that is, first electrode 11) into a bath (1) of an electrolyte solution in which a first oocyte and possibly one or more further oocytes can be immersed.

EXAMPLE 2

Baseline: When both the reference electrode (first electrode 11) and the microelectrode (12) are in electrical communication with electrolyte solution in the bath (1), the potential difference is zero millivolts (mV). In other words, a potential difference that can be measurable due to a probable compositional difference between the solution in the microelectrode and the electrolyte solution in the bath, can be set as a baseline potential difference (U0), and then taken as zero mV for simplifying consecutive voltage measurement recordings or monitoring (see FIG. 1A and/or FIG. 2A in conjunction with FIG. 3A and/or FIG. 3B).

EXAMPLE 3

Resistance of the second electrode: When a fixed amount of electric current (I) (e.g., 1 nanoAmper, 1 nA) is passed through the microelectrode (12), it causes a voltage deflection (that results in second electrode's potential difference (Ue) in mV) according to Ohm's Law (see below), which enables calculation of the electrode resistance (that is, resistance of the microelectrode, in M$\Omega$).

*Voltage (V) = Current (I) x Resistance (R)*

EXAMPLE 4

Oocyte potential difference and viability: Considering that cytoplasm of a viable oocyte contains a composition which can normally be different from that of the electrolyte in the bath (1); differences among the concentration of ions across the plasma membrane (510, 520) would result in a measurable potential difference between opposite sides of the plasma membrane. In a case where the oocyte lacks viability, said potential difference would be zero (non-barriered permeability). This knowledge allows to suggest a way of determining viability of an oocyte (51, 52).

Accordingly, referring back to FIG. 1C and/or FIG. 2C along with FIG. 3A or FIG. 3B; a respective one of the first oocyte (51) or one or more further oocytes (52) is engaged (e.g., impaled) by the microelectrode (12) in the absence of current (I=0) provision by the current source (71), and the membrane potential difference (that is, fourth potential difference for respective oocyte, referred to as Um, Um1, or Um2) can be recorded as the difference between inside and outside of the cell, i.e., between the reference electrode (11) and the microelectrode (12).

EXAMPLE 5

Cellular resistance: Referring back to FIG. 1D and/or FIG. 2D along with FIG. 3A or FIG. 3B; whilst the microelectrode (12) is engaged with the oocyte (first oocyte 51 and if applicable, second oocyte 52), the electric current ($I \neq 0$ Amperes) (that can be provided by the current source (71) is passed again (in other words, injected) through the microelectrode (12), and potential difference (first potential difference (U1) and if applicable, second potential difference (U2)) is measured. Deflection in voltage (|U1-Um1|, and if applicable, |U2-Um2|) , that is, difference (first potential difference change ($\Delta$U1) and if applicable, second potential difference change ($\Delta$U2)) between the membrane potential difference (fourth potential difference for the first oocyte (Um1), and if applicable, fourth potential difference for the second oocyte (Um2)) and the latter potential difference (first potential difference (U1) and if applicable, second potential difference (U2)) measured, enables calculation of a resistance value that corresponds to a sum of the second electrode's resistance (Re) (that is calculable from second electrode's potential difference Ue) and a cellular resistance; the latter reflecting a combination of (plasma-) membrane permeability ($R_m$) and cytoplasmic resistivity ($R_i$).

Oocytes have a spherical geometry. In order to calculate cellular resistance from the potential difference measurements within the context of the present disclosure, the following Ohm's law applied to spherical cells can be utilized (Equation 2 from Engel E., Barcilon V. and Eisenberg R.S., "Current-Voltage Curves Recorded by One Microelectrode" (The interpretation of current-voltage relations recorded from a spherical cell with a single microelectrode), pp. 384-403 (cf. page 385), Biophysical Journal, Volume 12, 1972):

$$Vi = I\ Rv + 8\ I\ Ri\ /\ (3\ \pi^2\ s) + [1 - e^{(-t\ /\ (Rm\ Cm))}]\ I\ Rm\ /\ (4\ \pi\ a^2)$$

Here, the first term represents the potential difference drop within the electrode (second electrode 12) and the other terms represent the parameters that such potential difference drop is correlated to. Vi represents the potential difference (in Volts) recorded inside the cell (respective oocyte 51 or 52) after the application of a step function of current ($I \neq 0$ Amperes), and can be taken as the first potential difference change ($\Delta$U1) or second potential difference change ($\Delta$U2), whichever is applicable. I (in Amperes) represents the amount of current applied. Rv (in Ohms) represents the second electrode's resistance (Re as referred to in the present specification) that can be considered relevant to tip geometry of the second electrode (12). Rm (in ohm-square centimeters) is the resistance of 1 square centimeter of membrane (510 or 520), that is herein used as a relative indicator for determining the extent of plasma membrane permeability. Cm (in farads per square centimeter) represents the capacitance of 1 square centimeter of membrane (510 or 520). Ri (in ohm-centimeters) represents the resistivity of the solution filling the cell (in other words, cytoplasmic resistance). The radius of the cell is shown with 'a' (in centimeters); whereas s (in centimeters) represents the inner radius of the opening (at the tip) of the microelectrode (12).

EXAMPLE 6

FIG. 5B shows a schematic section view relevant to the use of the second electrode (12) which can be selected as an intracellular sharp electrode within the context of the proposed technique, that can have a tip opening diameter of e.g., within a range between 0.01 micrometers and 0.1 micrometers, for electrical communication with intracellular compartment (that includes the cytoplasm) of a respective oocyte without mechanically damaging its membrane (510 or 520); and which can be arranged to provide an electrode resistance such as within the range between 20 and 50 MegaOhms as exemplified above. Also taking non-circular (e.g., oval or ellyptical) geometries at the tip opening into consideration, based on the exemplary tip opening diameter range indicated above, the second electrode (12) can comprise a tip opening with a flow sectional area within the range between $0.000025 \times \pi$ and $0.0025 \times \pi$ square micrometers ($\pi$ being the ratio between a circumference to a diameter of a circle).

EXAMPLE 7

FIG. 5C symbolizes subsequent electrical potential difference recordings corresponding to the two oocytes depicted in FIG. 5B, respectively. The (tip-) resistance of the second electrode (12) is measured before the cell membrane (510, 520) (which can be also referred to as plasma membrane, oocyte membrane or shortly, membrane) is penetrated according to deflection in voltage according to Ohm's law. While in the cell, membrane potential difference is recorded and the current injection is repeated. The recorded deflection in voltage, in this case, relates to the sum of input resistance and tip resistance. Input resistance is a measure of cell membrane permeability (that is combined with cytoplasmic resistance) and therefore, lower deflections in voltage corresponds to more permeable membranes, and vice versa. Validation of obtained data can be considered to require observation of similar tip resistance to that of the prior to impalement after the pipette (that is, second electrode 12) is withdrawn from the cell (that is, the respective oocyte) by indicating that the microelectrode remained intact throughout the recording. After these recordings, oocytes can be individually inseminated and cultured to correlate embryo development and oocyte-relevant electrophysiological parameters.

EXAMPLE 8

FIG. 6 relates to consecutive recordings for two denuded oocytes, that is, the oocytes are separated from their cumulus cells (521) investments. The denuded oocytes have extruded polar bodies and hence, they are likely at the Metaphase II stage. They have been impaled consecutively within the same culture media drop displaying similar membrane potential differences but different cellular resistances.

EXAMPLE 9

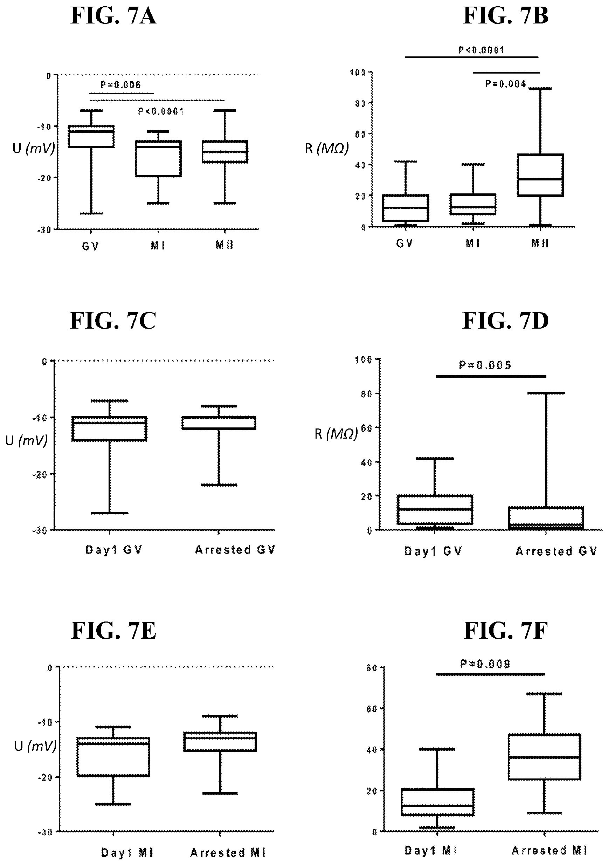

FIG. 7A and FIG. 7B show a proof of principle pilot data on denuded bovine oocytes showing changes in membrane potential difference (in milliVolts; mV) and input resistance (in MegaOhms; M$\Omega$) during maturation from Prophase 1 (GV) to Metaphase I (MI) and II (MII). Data are given as median (centre lines), 25% to 75% percentiles (boxes) and minimum/maximum values (bars). Statistical analysis has been performed using the GraphPad Prism software. Distribution of data have been assessed with D'Agostino & Pearson omnibus normality test. Differences among groups have been compared with parametric or non-parametric ANOVA and t-test or Mann-Whitney test, as appropriate.

EXAMPLE 10

FIG. 7C and FIG. 7D show comparisons in electrophysiological parameters of arrested oocytes at GV stage, to corresponding Day 1 oocytes. Oocytes, when they are aspirated from the ovaries, are at GV stage and progress to MI later during the day (Day 1). After 24 hours of in vitro culture, oocytes are expected to progress to MII, and those still displaying GV and/or MI stages have ceased their maturation, hence they are "arrested". Data are given as median (centre lines), 25% to 75% percentiles (boxes) and minimum/maximum values (bars). Statistical analysis has been performed using the GraphPad Prism software. Distribution of data have been assessed with D'Agostino & Pearson omnibus normality test. Differences among groups have been compared with parametric or non-parametric ANOVA and t-test or Mann-Whitney test, as appropriate.

EXAMPLE 11

FIG. 7E and FIG. 7F show comparisons in electrophysiological parameters of arrested oocytes at MI stage, to corresponding Day 1 oocytes. Data are given as median (centre lines), 25% to 75% percentiles (boxes) and minimum/-maximum values (bars). Statistical analysis has been performed using the GraphPad Prism software. Distribution of data have been assessed with D'Agostino & Pearson omnibus normality test. Differences among groups have been compared with parametric or non-parametric ANOVA and t-test or Mann-Whitney test, as appropriate.

EXAMPLE 12

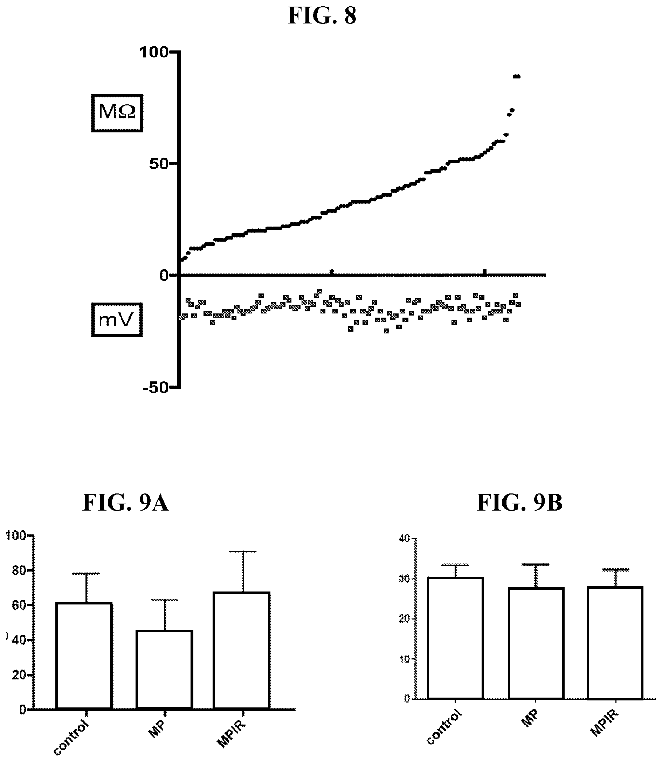

FIG. 8 shows electrical parameters profile of 111 metaphase II bovine oocytes (confirmed by DNA staining). Each dot represents one oocyte. Referring to ordinates, negative values represent membrane potential difference (in milliVolts), whereas positive values represent input (cellular) resistance (in megaOhms).

EXAMPLE 13

FIG. 9A shows proportion of mouse zygotes that yielded blastocysts after thawing (control, n=55), followed by cellular impalements without (MP, n=51) and with (MPIR, n=56) current injections ($P$=0.19, one-way ANOVA). Ordinates represent % blastocyst dev rate at 120 hours.

EXAMPLE 14

FIG. 9B in relation with FIG. 9A, shows total cell counts of blastocysts from the control (n=11), MP (n=7) and MPIR (n=6) groups (P=0.92, one-way ANOVA). The ordinates refer to total cell number of blastocysts.

EXAMPLE 15

Validation by re-measurement of second electrode's potential difference (Ue) (that is, potential difference relevant to resistance of the second electrode 12): After the second electrode (12) is withdrawn from the oocyte (51 and/or if applicable, 52), current injection (I≠0) can be repeated for Ue similarity to that at step-i, thereby ensuring that the second electrode (12) remained intact during the recording (see FIG. 3B: periods t52 and t54 for confirming the maintenance of Ue values that were respectively measured at periods t51 and t53).

The following part is considered useful for better understanding possible embodiments of the proposed method within the context of the main intended scope of the present application.

*A Tool to Monitor the Developmental Competency of the Oocyte:*

Embryo production, despite its widespread applications in medicine, biotechnology and agriculture, suffers from low feasibility because majority of in vitro created embryos are not capable of producing viable pregnancies. Such low efficacy is also observed in human assisted reproduction technology (ART), which is required to treat more than 70 million infertile couples globally. The proportion of live births to transferred embryos is 15.1% and only about 7% of the oocytes retrieved from the women produce an infant.

The capacity of the embryo to produce a viable pregnancy is mostly determined before fertilisation by the quality of oocyte. Besides providing the embryo half of the genetic material and nearly all membrane and cytoplasmic determinants, the oocyte is equipped with mechanisms that repair the damaged DNA, which the embryo may inherit from the sperm. These features, which determine the gamete's capacity to be fertilised and to support embryo development, are collectively referred as oocyte competency. It is probably mostly determined during foetal life due to inherent characteristics, which are susceptible to the effects of subsequently acquired features (e.g., hormonal imbalances, lifestyle, exposure to toxic substances etc.).

Oocytes attain competency during maturation through biological processes occurring within two cellular compartments; in the nucleus, genetic maturation ensures haploidy and it is completed in almost all species with fertilisation. The capacity of the oocyte to support the growth of the embryo, i.e., developmental competency, is attained during maturation of the cytoplasm through mechanisms that remain largely unknown. A prerequisite for this process to take place is maintenance of the interaction with the surrounding milieu, particularly with the attached cumulus cells. The communication within the cumulus-oocyte-complex (abbreviated as COC) established via the gap junction channels is maintained until the LH surge preceding ovulation, after when it is ceased, and resumption of meiosis is triggered. Artificial removal of cumulus cells from the antral follicle resumes meiosis, however, bypasses the 'oocyte capacitation', which is necessary for the attainment of a full developmental competence. In order to improve the synchrony between these processes, various experimental approaches have been tested with limited success. After ovulation, as a part of the process accompanying 'oocyte aging', the developmental competency begins to deteriorate if fertilisation does not occur within the 'optimal window' without an alteration in morphology.

Although the genetic composition of the gametes (and embryos) can be studied through various techniques, there are not any tools to predict the developmental competency of the oocyte. This causes utilisation of an alternative approach in ART, i.e., selection of embryos, because their capacity to yield a viable pregnancy can be predicted through relatively established criteria. The selection capacity is enhanced when a number of embryos are available at the cost of consuming many oocytes. This strategy contributes to the low efficacy observed with the embryo production technology and causes wastage of highly valuable biological material whose availability is limited during the female lifespan. It is likely that oocyte consumption can be minimised, and efficacy of ART can be improved through development of accurate and cost-effective real-time predictors of oocyte developmental capacity that are permissive in exhibition and tracing in live preparations without compromising cellular viability to allow subsequent utilisation for embryo generation.

The proposed technology hypothesizes that plasma membrane permeability changes and modifications in cytoplasmic characteristics occurring during maturation are correlated to the developmental competency of the oocyte.

Because these alterations are reflected to electrophysiological parameters (abbreviated as EP) of the oocyte as changes in the membrane potential difference (referred to as Um, or alternatively abbreviated as RMP) and the cellular or cytoplasmic input resistance (abbreviated as IR), they are proposed to enable monitoring the oocyte developmental competency in real time without compromising cellular viability or capacity to support embryo development.

The initial aims of the proposed technology can be considered to involve the following:

- To validate the proposed technique by monitoring EP during oocyte maturation,
- Assess the safety of the proposed technique by comparing embryo development in oocytes subjected to EP assays to controls,
- Determine the predictive value of EP assays in the assessment of developmental competency of denuded oocytes and COCs by establishing correlations to embryo development.

Referring to FIG. 5A, oocytes which have been stripped of (left) and enclosed (right) in their cumulus investments are secured in place with holding pipettes and impaled with sharp electrodes. Referring to FIG. 2B, after obtaining a stable membrane potential difference recording, current (e.g., 1 nanoAmper) is provided by a current source (71) and passed from the electrode (arrows, see FIG. 5B) into respective oocytes (51 and 52) with high (left, dotted line) or low (right, solid line) membrane permeability. FIG. 5C shows, EP recordings corresponding to the two oocytes in FIG. 5B. The tip resistance (second electrode's resistance Re) of the second electrode (12) is measured before the cell membrane (510 and 520, respectively) is penetrated (2) according to deflection in voltage according to Ohm's law. While in the respective oocyte (51 and 52), membrane potential difference (Um, also see Um1 and Um2 in FIG. 3A, FIG. 3B and FIG. 5C) is recorded and the current injection is repeated (also see t2 and t2' in FIG. 3A, or t21 and t22 within period O1, and t21' and t22' within period O2 in FIG. 3B). The recorded deflection in voltage (also see ΔU1 and ΔU2 in FIG. 3A, FIG. 3B and FIG. 5C), in this case, is caused by the sum of input resistance and second electrode's resistance (Re). Input resistance is a measure of cell membrane permeability and cytoplasmic resistance and therefore, lower deflections in voltage corresponds to more permeable membranes, and vice versa. Validation of obtained data requires observation of similar tip resistance to prior to impalement after the pipette is withdrawn from the cell (also see t52 after the period O1 and t54 after the period O2 in FIG. 3B) by indicating that the microelectrode (second electrode 12) remained intact throughout the recording. After recordings, oocytes can be individually inseminated and cultured to correlate embryo development and oocyte electrophysiological parameters.

Several methodological approaches have been suggested in prior art for evaluation of the developmental competence of oocytes, such as;

- conventional morphology assessment;
- Artificial Intelligence
- evaluation of cytoplasmic viscosity through observation of formation and persistence of the cytoplasmic funnel during ICSI,
- analysis of the spindle and the zona pellucida by polarizing microscopy,
- uptake of vital dyes to determine the activity of intracellular organelles,
- assessment of the metabolic turnover,
- molecular studies on genes and gene products obtained from the oocyte and the cumulus cells, and
- di-electrophoretic separation according to the gravity of the oocyte.

None of these prior art approaches produced a predictive tool, which fulfils all of the following criteria:

- objectivity and repeatability,
- real-time and continuous monitoring,
- allowing measurements from COCs,
- safety, non- toxicity and/or not disrupting the cell.

More recent approaches suggest the biomechanical properties of zygotes to correlate to their developmental competencies. However, the biomechanical parameters of the zygote are affected by the stiffness of the zona pellucida, which differs from that at the metaphase II oocyte due to hardening occurring with fertilisation. Furthermore, this approach can only be applied to oocytes after stripping them off from their surrounding cumulus cells, which may be necessary for the maintenance of the on-going mechanisms to regulate their developmental competency.

Information on the electrical characteristics of oocytes can also be obtained through application of the patch-clamp technique. However, the intracellular sharp electrode technique that can be employed within the context of the present application is superior because;

- a much more accurate measurement of membrane potential difference (Um, Um1, Um2) can be obtained through intracellular recordings,
- it minimises the risk that cells may be "dialyzed" with contents of the patch pipettes, which may gradually alter cellular properties during measurements,
- it can be applied without the need to remove the glycoprotein coat (zona pellucida) and the cumulus investments (see FIG. 5A, the further or second oocyte 52 at the right hand side, for significance of the presence of cumulus investments on oocyte maturity and attainment of competency).

This brings a unique opportunity of monitoring oocyte EP within COC (that is, physiological environment thereof) with minimal interruption, if any, to the mechanisms that attain the gamete its developmental competency. Accordingly, oocytes with or without cumulus cells differ in EP, although it is not known whether this variation is reflected to the competency of the gametes.

EXAMPLE 16

The proposed EP has been applied to bovine oocytes and detected EP differences among individual gametes at similar genetic maturation stages (FIG. 8) and also among immature and in vitro matured oocytes (FIG. 7A, b). The spectrum of EP variation observed among individual metaphase II oocytes (as confirmed by DNA staining) was wider in IR as compared to RMP (FIG. 8). The potential significance of EP variations to predict the developmental competence of individual oocytes remains to be assessed. Application of the proposed EP to mouse zygotes also did not compromise subsequent embryo development (FIG. 9).

Genetically mature (that is, metaphase II, abbreviated as MII) bovine oocytes displayed a significantly hyperpolarized (that is, more negative) RMP and a significantly higher IR than those in the immature (germinal vesicle) stage (FIG. 7A,b) indicating that plasma membrane permeability decreased and the resistivity of the cytoplasm increased with maturation of the bovine oocytes. Significant EP differences have also been observed between genetically maturing oocytes and those that have been arrested (FIG. 7C to FIG. 7F).

The following manipulations associated with the technique proposed herein may be considered to impose safety concerns, yet the examples provided in the present disclosure show that said safety concerns are overcome:

- no mechanical damage is imposed through impalement of the oocyte, and
- injection of an electrical current into the cell does not cause any problem. Impalement of oocytes with the ICSI technique is a routine clinical practise in ART since early 1990s.

The proposed technique does not cause a mechanical damage that exceeds that of ICSI because the diameter of the tip opening of ICSI pipettes is approximately 6 $\mu$m to accommodate the sperm, which is significantly wider than the 0.1 $\mu$m (or less) than the microelectrodes used in the proposed technique. Accordingly, loss of viability has been observed in less than 1% of oocytes during experiments. Furthermore, RMP monitoring of human oocytes during ICSI has earlier been practised to ascertain the intracellular localisation of the injection pipette.

The safety of the approach has been tested by comparing embryo development in thawed mouse zygotes (B6C3F1 X B6D2F1, EmbryoTech Laboratories) subjected to the proposed technique with the controls. Experiments have been repeated 6 times with the same batch and each set included:

- control Group, zygotes were cultured after thawing,

- membrane potential difference (Um, orMP) Group, zygotes were impaled to measure RMP and cultured, and

- Input Resistance (MPIR) Group, zygotes were impaled, and an electrical current has been passed to measure both RMP and IR, respectively, and cultured.

The power calculation estimated that a minimal of 51 zygotes was required in each group. A total of 161 cryopreserved zygotes have been cultured in PrimoVision dishes for 120 hours. Embryo development has been photographed and a subgroup of blastocysts (n=24) has been differentially stained for total cell counts. The results showed that the proposed technique did not compromise embryo viability and total cell counts remained similar among groups (see FIG. 9A and FIG. 9B).

The proposed technology can find implications in; (i) basic science: exploration the membrane alterations leading to attainment of competency in mammalian oocytes and embryos using a combination of cell and molecular biology techniques and electrophysiology, and (ii) clinical sciences: implementation of an "EP-based sequential analysis model" yielding an integrated score of developmental competence in oocytes and early-stage embryos.

Follow up proposed technology will incorporate studies that extend knowledge in membrane properties from oocytes to preimplantation embryos. The onset and extent of intercellular communication in the early stages of development will be correlated to embryo Viability. Cells in the preimplantation embryo communicate through gap junctions, however their significance to subsequent development is not clear. Although the embryo is electrically coupled as early as the 2-cell stage, intercellular communication has been claimed responsible for cavitation and blastocyst formation. A recent time-lapse study indicated a correlation with the dimensions of intercellular contact between blastomeres of a 4-cell embryo and implantation capacity. This proposed technology will aim to profile the intercellular communication in the embryo at the molecular, morphological and physiological level and assess potential correlations to embryo Viability.

Substantiation of the animal data with the human studies is expected to yield an industrial product that can be utilised in medicine, biomedicine and in food industry to develop novel approaches for increasing efficiency in IVM, artificial gamete production technology and clinical outcomes in ART.

**Claims**

1. A method for benchmarking of mammalian oocytes in terms of their comparative extents of potential for supporting a subsequent embryo development relative to one another; wherein the method comprises the following steps:

    step-A)    determination of relative extents of respective plasma membrane permeabilities for electric currents, of a first oocyte (51) and a second oocyte (52) with regard to one another, based on one or more measurements of relative extents of one or more electrical parameters of the first oocyte (51) and second oocyte (52),
        and

    step-B)    sorting the first oocyte (51) and the second oocyte (52) in accordance with their relative extents of respective plasma membrane permeabilities, such that one of the first oocyte (51) and the second oocyte (52) with a relatively lower plasma-membrane permeability is bench marked as having a relatively greater extent of potential for supporting the subsequent embryo development,

**characterized in that** the step-A comprises the following steps:

    i) placement of the first oocyte (51) Into the electrolyte bath (1);
    II) when the first electrode (11) is immersed into the electrolyte bath (1), and the second electrode (12) is engaged to the first oocyte (51), provision of the electrical current (I) between the first electrode (11) and second electrode (12) by a current source (71), such that the electrical current flows through the electrolyte and the membrane (510) of the first oocyte (51); measuring and recording a first potential difference change ($\Delta U1$) value in relation with the first oocyte (51);
    iii) placement of the second oocyte (52) into the electrolyte bath (1);
    Iv) when the first electrode (11) is immersed into the electrolyte bath (1), and the second electrode (12) is engaged to the second oocyte (52), provision of the electrical current (I) between the first electrode (11) and second electrode (12) by the current source (71), such that the electrical current flows through the electrolyte and the membrane (520) of the second oocyte (52); measuring and recording a second potential difference change ($\Delta U2$) value in relation with the second oocyte (52);

    and the step-B comprises the following:
    if the first potential difference change ($\Delta U1$) is smaller than the second potential difference change ($\Delta U2$), benchmarking the second oocyte (52) as having a relatively greater extent of potential for supporting subsequent embryo development; or if the first potential difference change ($\Delta U1$) is greater than the second potential difference change ($AU2$), benchmarking the second oocyte (52) as having a relatively lower extent of potential for supporting subsequent embryo development.

2. The method according to claim 1, comprising selection of electrical resistance as the one or more electrical parameters.

3. The method according to claim 2, comprising calculation of the electrical resistance from the potential difference measurements.

4. The method according to any of claims 1 to 3, wherein the step ii comprises calculation of a first resistance value based on the first potential difference change ($\Delta U1$), and attributing the first resistance value as a membrane resistance of the first oocyte (51); and the step iv comprises calculation of a second resistance value based on the second potential difference change ($\Delta U2$), and attributing the second resistance value as a membrane resistance of the second oocyte (52).

5. The method according to claim 4; wherein the step-B comprises the following:

   B1) determining which one of the first resistance value and second resistance value is relatively higher;
   B2) benchmarking the first oocyte (51) as having a relatively greater extent of potential for supporting subsequent embryo development, If the first resistance value Is higher than the second resistance value; or benchmarking the second oocyte (52) as having a relatively greater extent of potential for supporting subsequent embryo development, if the second resistance value is higher than the first resistance value.

6. The method according to any of claims 1 to 5, wherein the method comprises the following step-x prior to the step-ii:
   x) measurement of a potential difference between the first electrode (11) and the second electrode (12) that are immersed into the electrolyte bath (1) in the absence of the electrical current (I) between the first electrode (11) and second electrode (12) by the current source (71), wherein the second electrode (12) is not engaged with any of the first oocyte (51) or second oocyte (52); thereby obtaining a baseline potential difference (U0) value.

7. The method according to claim 6, wherein the step ii and/or step iv are followed by extraction of the second electrode from the first oocyte (51) or second oocyte (52), and measuring potential difference for confirming whether the baseline potential difference (U0) is maintained in the absence of the electrical current (I) between the first electrode (11) and second electrode (12) by the current source (71); and confirming that the second electrode (12) is still functional if the potential difference among consecutive measurements does not deviate more than 10% from an initial value of the baseline potential difference (U0).

8. The method according to any of claims 1 to 7, comprising the following:

   when the second electrode (11) is engaged to the first oocyte (51), measuring a fourth potential difference (Um1) for the first oocyte (51) in the absence of electrical current (I) feed by the current source (71) between the first electrode (11) and second electrode (12); checking whether a difference between the baseline potential difference (U0) and fourth potential difference (Um1) for the first oocyte (51) is different from zero, for confirming an initial viability status for the first oocyte (51); and/or
   when the second electrode (11) is engaged to the second oocyte (52), measuring the fourth potential difference (Um2) for the second oocyte (52) in the absence of electrical current (I) feed by the current source (71) between the first electrode (11) and second electrode (12); checking whether a difference between the baseline potential difference (U0) and fourth potential difference (Um2) for the second oocyte (52) is different from zero, for confirming an initial viability status for the second oocyte (52).

9. The method according to claim 8; comprising the following:

   checking whether the difference between the baseline potential difference (U0) and fourth potential difference (Um1) for the first oocyte (51) does not deviate more than 10% on the basis of an initial absolute value of said difference, at the end of a first duration for the first oocyte (t1), for confirming a viability status for the first oocyte (51); and/or
   checking whether the difference between the baseline potential difference (U0) and fourth potential difference (Um2) for the second oocyte (52) does not deviate more than 10% on the basis of an initial absolute value of said difference, at the end of a respective first duration for the second oocyte (t1'), for confirming a viability status for the second oocyte (52).

10. The method according to any of claims 1 to 9; comprising removal of one or more cumulus cells (521) from the first oocyte (51) and/or second oocyte (52) prior to the step-A.

11. The method according to any of claims 1 to 10; wherein the current source (71) is arranged to provide an electrical current of 1 nanoAmper.

12. The method according to any of claims 1 to 11, wherein the second electrode (12) is arranged to have an electrode resistance within the range between 20 MegaOhms and 50 MegaOhms.

13. The method according to any of claims 1 to 12; wherein the second electrode (12) comprises a tip opening with a flow sectional area within the range between 0.000025×π and 0.0025×π square micrometres.

14. A method for benchmarking of mammalian oocytes in terms of their extents of potential for supporting a subsequent embryo development; wherein the method comprises the following steps;

    step-A)    determination of a relative extent of a plasma membrane permeability of a first oocyte (51) for electric currents, over a first resistance value in relation with the first oocyte (51), based on one or more measurements which include a provision of an electrical current (I) between a first electrode (11) and a second electrode (12) which is engaged to the first oocyte (51); and

    step-B)    determining whether the first resistance value is higher than a pre-determined reference value; and if the first resistance value is greater than the reference value, benchmarking the first oocyte (51) as having a sufficient extent of potential for supporting subsequent embryo development,

**characterized in that** the step-A comprises the following steps:

- placement of the first oocyte (51) into an electrolyte bath (1);
- bringing the first electrode (11) into electrical communication with the electrolyte bath (1), and bringing the second electrode (12) into electrical communication with an intracellular medium of the first oocyte (51), providing the electrical current (I) between the first electrode (11) and second electrode (12) by a current source (71), such that the electrical current flows through a membrane (510) of the first oocyte (51); measuring and recording a first potential difference change (ΔU1) value In relation with the first oocyte (51); calculation of the first resistance value based on the first potential difference change (ΔU1).

## Patentansprüche

1. Verfahren zur Benchmarkierung von Säugeroozyten anhand ihrer Vergleichsausmaße von Potential zur Unterstützung einer nachfolgenden Embryoentwicklung relativ zueinander; wobei das Verfahren umfasst folgende Schritte:

    Schritt-A)    Bestimmen von relativen Ausmaßen jeweiliger Plasmamembranpermeabilitaten für elektrische Ströme, eines ersten Oozyten (51) und eines zweiten Oozyten (52) zueinander, basierend auf einer oder mehreren Messungen von relativen Ausmaßen eines oder mehrerer elektrischer Parameter des ersten Oozyten (51) und des zweiten Oozyten (52), und

    Schritt-B)    Sortieren der ersten Oozyte (51) und der zweiten Oozyte (52) in Übereinstimmung mit ihren relativen Ausmaßen der jeweiligen Plasmamembranpermeabilitäten, so dass eine der ersten Oozyte (51) und der zweiten Oozyte (52) mit einer relativ niedrigeren Plasmamembranpermeabilitat benchmarkiert wird als mit einem relativ größeren Ausmaß an Potential zum Unterstützen der nachfolgenden Embryoentwicklung;

**dadurch gekennzeichnet, dass** der Schritt-A die folgenden Schritte umfasst:

i) Einbringen des ersten Oozyten (51) in das Elektrolytbad (1);
ii) wenn die erste Elektrode (11) in das Elektrolytbad (1) eingetaucht ist und die zweite Elektrode (12) mit dem ersten Oozyt (51) in Eingriff steht, Bereitstellen des elektrischen Stroms (I) zwischen der ersten Elektrode (11) und der zweiten Elektrode (12) durch eine Stromquelle (71), so dass der elektrische Strom durch den Elektrolyten und die Membran (510) des ersten Oozyten (51) fließt; Messen und Aufzeichnen eines Werts einer ersten Potenzielldifferenzänderung (ΔU1) in Bezug auf den ersten Oozyt (51);
iii) Einbringen des zweiten Oozyten (52) in das Elektrolytbad (1);
iv) wenn die erste Elektrode (11) in das Elektrolytbad (1) eingetaucht ist und die zweite Elektrode (12) mit dem zweiten Oozyt (52) in Eingriff steht, Bereitstellen des elektrischen Stroms (I) zwischen der ersten Elektrode (11) und der zweiten Elektrode (12) durch die Stromquelle (71), so dass der elektrische Strom durch den Elektrolyten und die Membran (520) des zweiten Oozyten (52) fließt; Messen und Aufzeichnen eines Werts einer zweiten Potentialdifferenzänderung (ΔU2) in Bezug auf den zweiten Oozyt (52);

und der Schritt-B Folgendes umfasst:

wenn die erste Potentialdifferenzänderung (ΔU1) kleiner als die zweite Potentialdifferenzänderung (ΔU2) ist, Benchmarkieren des zweiten Oozyten (52) mit einem relativ größeren Ausmaß an Potential zur Unterstützung einer nachfolgenden Embryoentwicklung; oder wenn die erste Potentialdifferenzänderung (ΔU1) grösser als die zweite Potentialdifferenzänderung (ΔU2) ist, Benchmarkieren des zweiten Oozyten (52) mit einem relativ geringeren Ausmaß an Potential zur Unterstützung einer nachfolgenden Embryoentwicklung.

2. Verfahren nach Anspruch 1, umfassend die Auswahl des elektrischen Widerstands als den einen oder die mehreren elektrischen Parameter.

3. Verfahren nach Anspruch 2, umfassend die Berechnung des elektrischen Widerstands aus den Potentialdifferenzmessungen.

4. Das Verfahren nach einem der Ansprüche 1 bis 3, wobei der Schritt ii ein Berechnen eines ersten Widerstandswerts basierend auf der ersten Potentialdifferenzänderung (ΔU1) und ein Zuordnen des ersten Widerstandswerts als Membranwiderstand des ersten Oozyten (51) umfasst; und der Schritt iv ein Berechnen eines zweiten Widerstandswerts basierend auf der zweiten Potentialdifferenzänderung (ΔU2) und ein Zuordnen des zweiten Widerstandswerts als Membranwiderstand des zweiten Oozyten (52).

5. Verfahren nach Anspruch 4, wobei der Schritt-B Folgendes umfasst:

   B1)   Bestimmen, welcher von dem ersten Widerstandswert und dem zweiten Widerstandswert relativ höher ist;

   B2)   Benchmarkieren des ersten Oozyten (51) mit einem relativ größeren Ausmaß an Potential zur Unterstützung der nachfolgenden Embryoentwicklung, wenn der erste Widerstandswert höher als der zweite Widerstandswert ist; oder Benchmarkieren des zweiten Oozyten (52) mit einem relativ größeren Ausmaß an Potential zur Unterstützung der nachfolgenden Embryoentwicklung, wenn der zweite Widerstandswert höher als der erste Widerstandswert ist.

6. Verfahren nach einem der Ansprüche 1 bis 5, wobei das Verfahren den folgenden Schritt-x vor dem Schritt ii:
   x) Messung einer Potentialdifferenz zwischen der ersten Elektrode (11) und der zweiten Elektrode (12), die in Abwesenheit des elektrischen Stroms (I) zwischen der ersten Elektrode (11) und der zweiten Elektrode (12) durch die Stromquelle (71) in das Elektrolytbad (1) eingetaucht sind, wobei die zweite Elektrode (12) nicht mit der ersten Oozyte (51) oder der zweiten Oozyte (52) in Eingriff steht; wodurch der Wert einer Grundlinienpotentialdifferenz (U0) erhalten wird.

7. Verfahren nach Anspruch 6, wobei dem Schritt ii und/oder Schritt iv ein Extrahieren der zweiten Elektrode aus dem ersten Oozyt (51) oder dem zweiten Oozyt (52) und ein Messen einer Potentialdifferenz zum Bestätigen, ob die Grundlinienpotentialdifferenz (U0) in Abwesenheit des elektrischen Stroms (I) zwischen der ersten Elektrode (11) und der zweiten Elektrode (12) durch die Stromquelle (71) aufrechterhalten wird; und bestätigen, dass die zweite Elektrode (12) noch funktionsfähig ist, wenn die Potentialdifferenz zwischen aufeinanderfolgenden Messungen nicht mehr als 10% von einem Anfangswert der Grundlinienpotentialdifferenz (U0).

8. Verfahren nach einem der Ansprüche 1 bis 7, umfassend:
   Wenn die zweite Elektrode (11) mit der ersten Oozyte (51) in Eingriff steht, Messen einer vierten Potentialdifferenz (Um1) für die erste Oozyte (51) in Abwesenheit eines elektrischen Stroms (I), der von der Stromquelle (71) zwischen der ersten Elektrode (11) und der zweiten Elektrode (12) gespeist wird; prüfen, ob eine Differenz zwischen der Grundlinienpotentialdifferenz (U0) und der vierten Potentialdifferenz (Um1) für die erste Oozyte (51) von Null verschieden ist, zum Bestätigen eines anfänglichen Lebensfähigkeitsstatus für die erste Oozyte (51); und/oder Wenn die zweite Elektrode (11) mit dem zweiten Oozyt (52) in Eingriff steht, Messen der vierten Potentialdifferenz (Um2) für den zweiten Oozyt (52) in Abwesenheit eines elektrischen Stroms (I), der von der Stromquelle (71) zwischen der ersten Elektrode (11) und der zweiten Elektrode (12) gespeist wird; prüfen, ob eine Differenz zwischen der Grundlinienpotentialdifferenz (U0) und der vierten Potentialdifferenz (Um2) für den zweiten Oozyt (52) von Null verschieden ist, zum Bestätigen eines anfänglichen Lebensfähigkeitsstatus für den zweiten Oozyt (52).

9. Verfahren nach Anspruch 8; umfassend das Folgende:
   Prüfen, ob die Differenz zwischen der Grundlinienpotentialdifferenz (U0) und der vierten Potentialdifferenz (Um1) für die erste Oozyte (51) nicht mehr als 10% abweicht, basierend auf einem anfänglichen Absolutwert der Differenz, am Ende einer ersten Dauer für die erste Oozyte (t1), zum Bestätigen eines Lebensfähigkeitsstatus für die erste Oozyte (51); und/oder prüfen, ob die Differenz zwischen der Grundlinienpotentialdifferenz (U0) und der vierten Potentialdifferenz (Um2) für die zweite Oozyte (52) nicht mehr als 10% abweicht, basierend auf einem anfänglichen Absolutwert der Differenz, am Ende einer jeweiligen ersten Dauer für die zweite Oozyte (t1'), zum Bestätigen eines Lebensfähigkeitsstatus für die zweite Oozyte (52).

10. Verfahren nach einem der Ansprüche 1 bis 9; umfassend das Entfernen einer oder mehrerer Kumuluszellen (521) aus der ersten Oozyte (51) und/oder der zweiten Oozyte (52) vor dem Schritt-A.

11. Verfahren nach einem der Ansprüche 1 bis 10; wobei die Stromquelle (71) angeordnet ist, um einen elektrischen Strom von 1 Nanoampere bereitzustellen.

12. Verfahren nach einem der Ansprüche 1 bis 11, wobei die zweite Elektrode (12) so angeordnet ist, dass sie einen Elektrodenwiderstand im Bereich zwischen 20 Megaohm und 50 Megaohm aufweist.

13. Verfahren nach einem der Ansprüche 1 bis 12; wobei die zweite Elektrode (12) eine Spitzenöffnung mit einer Strömungsschnittfläche im Bereich zwischen $0,000025 \times \pi$ und $0,0025 \times \pi$ Quadratmikrometer umfasst.

14. Verfahren zur Benchmarkierung von Säuger-Oozyten anhand ihrer Potentialausmaße zur Unterstützung einer nachfolgenden Embryoentwicklung; wobei das Verfahren die folgenden Schritte umfasst;

    Schritt-A)  Bestimmen eines relativen Ausmaßes einer Plasmamembranpermeabilitat eines ersten Oozyten (51) für elektrische Ströme über einen ersten Widerstandswert in Bezug auf den ersten Oozyten (51), Basierend auf einer oder mehreren Messungen, die eine Bereitstellung eines elektrischen Stroms (I) zwischen einer ersten Elektrode (11) und einer zweiten Elektrode (12) umfassen, die mit dem ersten Oozyten (51); und

    Schritt-B)  Bestimmen, ob der erste Widerstandswert höher als ein vorbestimmter Referenzwert ist; und wenn der erste Widerstandswert grösser als der Referenzwert ist, Benchmarkieren des ersten Oozyten (51) mit einem ausreichenden Ausmaß an Potential zum Unterstützen der nachfolgenden Embryoentwicklung,

    **dadurch gekennzeichnet, dass** der Schritt-A die folgenden Schritte umfasst:

    - Einbringen der ersten Oozyte (51) in ein Elektrolytbad (1);
    - Bringen der ersten Elektrode (11) in elektrische Kommunikation mit dem Elektrolytbad (1), und Bringen der zweiten Elektrode (12) in elektrische Kommunikation mit einem intrazellulären Medium des ersten Oozyten (51); Bereitstellen des elektrischen Stroms (I) zwischen der ersten Elektrode (11) und der zweiten Elektrode (12) durch eine Stromquelle (71), so dass der elektrische Strom durch eine Membran (510) des ersten Oozyten (51) fließt; Messen und Erfassen des Werts einer ersten Potentialdifferenzänderung ($\Delta$U1) in Bezug auf den ersten Oozyten (51); Berechnen des ersten Widerstandswerts basierend auf der ersten Potentialdifferenzänderung ($\Delta$U1).

## Revendications

1. Procédé de référenciation d'ovocytes de mammifère en termes de leurs étendues comparatives de potentiel pour supporter un développement embryonnaire ultérieur l'un par rapport à l'autre ; dans lequel le procédé comprend les étapes suivantes :

   étape-A) détermination d'étendues relatives de perméabilités de membrane plasmatique respectives pour des courants électriques, d'un premier ovocyte (51) et d'un second ovocyte (52) l'un par rapport à l'autre, sur la base d'une ou de plusieurs mesures d'étendues relatives d'un ou de plusieurs paramètres électriques du premier ovocyte (51) et du second ovocyte (52),
   et
   étape-B) tri du premier ovocyte (51) et du second ovocyte (52) en fonction de leurs étendues relatives de perméabilités de membrane plasmatique respectives, de sorte que l'un parmi le premier ovocyte (51) et le second ovocyte (52) avec une perméabilité de membrane plasmatique relativement plus faible soit référencé comme ayant une étendue relativement plus grande de potentiel pour supporter le développement embryonnaire ultérieur,

**caractérisé en ce que** l'étape-A comprend les étapes suivantes :

i) placement du premier ovocyte (51) dans le bain électrolytique (1) ;

ii) lorsque la première électrode (11) est immergée dans le bain électrolytique (1), et que la seconde électrode (12) est mise en prise avec le premier ovocyte (51), fourniture du courant électrique (I) entre la première électrode (11) et la seconde électrode (12) par une source de courant (71), de sorte que le courant électrique circule à travers l'électrolyte et la membrane (510) du premier ovocyte (51) ; mesure et enregistrement d'une première valeur de changement de différence de potentiel ($\Delta$U1) par rapport au premier ovocyte (51) ;

iii) placement du second ovocyte (52) dans le bain électrolytique (1) ;

iv) lorsque la première électrode (11) est immergée dans le bain électrolytique (1), et que la seconde électrode (12) est mise en prise avec le second ovocyte (52), fourniture du courant électrique (I) entre la première électrode (11) et la seconde électrode (12) par la source de courant (71), de sorte que le courant électrique circule à travers l'électrolyte et la membrane (520) du second ovocyte (52) ; mesure et enregistrement d'une seconde valeur de changement de différence de potentiel ($\Delta$U2) par rapport au second ovocyte (52) ;

et l'étape-B comprend l'élément suivant :

si le premier changement de différence de potentiel ($\Delta$U1) est plus petit que le second changement de différence de potentiel ($\Delta$U2), référenciation du second ovocyte (52) comme ayant une étendue de potentiel relativement plus grande pour supporter le développement embryonnaire ultérieur ; ou si le premier changement de différence de potentiel ($\Delta$U1) est plus grand que le second changement de différence de potentiel ($\Delta$U2), référenciation du second ovocyte (52) comme ayant une étendue de potentiel relativement plus faible pour supporter le développement embryonnaire ultérieur.

2. Procédé selon la revendication 1, comprenant la sélection d'une résistance électrique comme les un ou plusieurs paramètres électriques.

3. Procédé selon la revendication 2, comprenant le calcul de la résistance électrique à partir des mesures de différence de potentiel.

4. Procédé selon l'une quelconque des revendications 1 à 3, dans lequel l'étape ii comprend le calcul d'une première valeur de résistance sur la base du premier changement de différence de potentiel ($\Delta$U1), et l'attribution de la première valeur de résistance en tant que résistance de membrane du premier ovocyte (51) ; et l'étape iv comprend le calcul d'une seconde valeur de résistance sur la base du second changement de différence de potentiel ($\Delta$U2), et l'attribution de la seconde valeur de résistance en tant que résistance de membrane du second ovocyte (52).

5. Procédé selon la revendication 4 ; dans lequel l'étape B comprend les étapes suivantes :

B1) détermination de laquelle parmi la première valeur de résistance et la seconde valeur de résistance est relativement plus élevée ;

B2) référenciation du premier ovocyte (51) comme ayant une étendue relativement plus grande de potentiel pour supporter le développement embryonnaire ultérieur, si la première valeur de résistance est plus élevée que la seconde valeur de résistance ; ou référenciation du second ovocyte (52) comme ayant une étendue relativement plus grande de potentiel pour supporter le développement embryonnaire ultérieur, si la seconde valeur de résistance est plus élevée que la première valeur de résistance.

6. Procédé selon l'une quelconque des revendications 1 à 5, dans lequel le procédé comprend l'étape-x suivante préalable à l'étape-ii :

x) mesure d'une différence de potentiel entre la première électrode (11) et la seconde électrode (12) qui sont immergées dans le bain électrolytique (1) en l'absence de courant électrique (I) entre la première électrode (11) et la seconde électrode (12) par la source de courant (71), dans lequel la seconde électrode (12) n'est mise en prise avec aucun du premier ovocyte (51) ou du second ovocyte (52) ; obtenant ainsi une valeur de différence de potentiel de référence (U0).

7. Procédé selon la revendication 6, dans lequel l'étape ii et/ou l'étape iv sont suivies d'une extraction de la seconde électrode à partir du premier ovocyte (51) ou du second ovocyte (52), et d'une mesure de la différence de potentiel pour confirmer si la différence de potentiel de référence (U0) est maintenue en l'absence du courant électrique (I) entre la première électrode (11) et la seconde électrode (12) par la source de courant (71) ; et la confirmation que la seconde électrode (12) est toujours fonctionnelle si la différence de potentiel entre des mesures consécutives ne s'écarte pas de plus de 10 % d'une valeur initiale de la différence de potentiel de référence (U0).

8. Procédé selon l'une quelconque des revendications 1 à 7, comprenant les éléments suivants :

   lorsque la seconde électrode (11) est mise en prise avec le premier ovocyte (51), la mesure d'une quatrième différence de potentiel (Um1) pour le premier ovocyte (51) en l'absence d'alimentation de courant électrique (I) par la source de courant (71) entre la première électrode (11) et la seconde électrode (12) ; le fait de vérifier si une différence entre la différence de potentiel de référence (U0) et la quatrième différence de potentiel (Um1) pour le premier ovocyte (51) est différente de zéro, pour confirmer un état de viabilité initial pour le premier ovocyte (51) ; et/ou
   lorsque la seconde électrode (11) est mise en prise avec le second ovocyte (52), la mesure de la quatrième différence de potentiel (Um2) pour le second ovocyte (52) en l'absence d'alimentation de courant électrique (I) par la source de courant (71) entre la première électrode (11) et la seconde électrode (12) ; le fait de vérifier si une différence entre la différence de potentiel de référence (U0) et la quatrième différence de potentiel (Um2) pour le second ovocyte (52) est différente de zéro, pour confirmer un état de viabilité initial pour le second ovocyte (52).

9. Procédé selon la revendication 8, comprenant les éléments suivants :

   le fait de vérifier si la différence entre la différence de potentiel de référence (U0) et la quatrième différence de potentiel (Um1) pour le premier ovocyte (51) ne s'écarte pas de plus de 10 % sur la base d'une valeur absolue initiale de ladite différence, à la fin d'une première durée pour le premier ovocyte (t1), pour confirmer un état de viabilité pour le premier ovocyte (51) ; et/ou
   le fait de vérifier si la différence entre la différence de potentiel de référence (U0) et la quatrième différence de potentiel (Um2) pour le second ovocyte (52) ne s'écarte pas de plus de 10 % sur la base d'une valeur absolue initiale de ladite différence, à l'issue d'une première durée respective pour le second ovocyte (t1'), pour confirmer un état de viabilité pour le second ovocyte (52).

10. Procédé selon l'une quelconque des revendications 1 à 9, comprenant l'élimination d'une ou de plusieurs cellules de cumulus (521) du premier ovocyte (51) et/ou du second ovocyte (52) avant l'étape-A.

11. Procédé selon l'une quelconque des revendications 1 à 10, dans lequel la source de courant (71) est agencée pour fournir un courant électrique de 1 nanoampère.

12. Procédé selon l'une quelconque des revendications 1 à 11, dans lequel la seconde électrode (12) est agencée pour présenter une résistance d'électrode dans la plage comprise entre 20 mégohms et 50 mégohms.

13. Procédé selon l'une quelconque des revendications 1 à 12, dans lequel la seconde électrode (12) comprend une ouverture de pointe avec une surface de section de flux dans la plage comprise entre $0,000025 \times \pi$ et $0,0025 \times \pi$ micromètres carrés.

14. Procédé de référenciation d'ovocytes de mammifère en termes de leurs étendues de potentiel pour supporter un développement embryonnaire ultérieur, dans lequel le procédé comprend les étapes suivantes :

   étape-A) détermination d'une étendue relative de perméabilité d'une membrane plasmatique d'un premier ovocyte (51) pour des courants électriques, sur une première valeur de résistance par rapport au premier ovocyte (51),
   sur la base d'une ou de plusieurs mesures qui comportent la fourniture d'un courant électrique (I) entre une première électrode (11) et une seconde électrode (12) qui est mise en prise avec le premier ovocyte (51) ; et
   étape-B) détermination du fait que la première valeur de résistance est plus élevée ou non qu'une valeur de référence prédéterminée ; et si la première valeur de résistance est plus élevée que la valeur de référence, la référenciation du premier ovocyte (51) comme ayant une étendue suffisante de potentiel pour supporter le développement embryonnaire ultérieur,

**caractérisé en ce que** l'étape-A comprend les étapes suivantes :

- le placement du premier ovocyte (51) dans un bain électrolytique (1) ;
- le fait d'amener la première électrode (11) en communication électrique avec le bain électrolytique (1), et d'amener la seconde électrode (12) en communication électrique avec un milieu intracellulaire du premier ovocyte (51), la fourniture du courant électrique (I) entre la première électrode (11) et la seconde électrode (12) par une source de courant (71), de sorte que le courant électrique circule à travers une membrane (510) du premier ovocyte (51) ; la mesure et l'enregistrement d'une première valeur de différence de potentiel ($\Delta U1$) par rapport au premier ovocyte (51) ; le calcul de la première valeur de résistance sur la base du premier changement de différence de potentiel ($\Delta U1$).

## FIG. 1A

## FIG. 1B

# FIG. 1C

100

7

72

70

71

I=0

72

51

12

510

11

1

# FIG. 1D

100

7

72

70

71

I≠0

72

51

12

510

11

1

## FIG. 2A

## FIG. 2B

## FIG. 2C

## FIG. 2D

## FIG. 3A

## FIG. 3B

# FIG. 4

# FIG. 5A

# FIG. 5B

## FIG. 5C

## FIG. 6

**FIG. 7A**

**FIG. 7B**

**FIG. 7C**

**FIG. 7D**

**FIG. 7E**

**FIG. 7F**

## FIG. 8

## FIG. 9A

## FIG. 9B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020077244 A1

**Non-patent literature cited in the description**

- **PALAY et al.** *Lab on a Chip,* 2024, vol. 24, 3909-3929

- **ENGEL E.** ; **BARCILON V.** ; **EISENBERG R.S.** Current-Voltage Curves Recorded by One Microelectrode. *The interpretation of current-voltage relations recorded from a spherical cell with a single microelectrode,* 384-403
- *Biophysical Journal,,* 1972, vol. 12